# EUROPEAN PATENT APPLICATION

(11) **EP 4 018 994 A1**
(43) Date of publication of application: **29.06.2022**
(21) Application number: 21176613.4
(22) Date of filing: 28.05.2021
(51) Int. Cl.: A61K 8/31, A61K 8/36, A61K 8/67, A61K 8/92, A61Q 19/00

(54) **COMPOSITION COMPRISING OILS, FREE FATTY ACIDS AND SQUALENE**

(71) Applicant: Clariant International Ltd, 4132 Muttenz (CH)
(72) Inventor: MINOGGIO, Isabelle, 65926 Frankfurt a.M. (DE); ZMIRIC, Aleksandra, 20037 Palazzolo Milanese (IT); WENSKI, Svenja, 65926 Frankfurt a.M. (DE)
(74) Representative: Kampen, Daniela

(57) **Abstract**

The present invention relates to a composition comprising a triglyceride-containing oil, jojoba oil, a free fatty acid or a salt thereof, and squalene. The present invention also relates to the use of such a composition as a skin conditioning agent.

## Description

The present invention relates to a composition comprising a triglyceride-containing oil, jojoba oil, a free fatty acid or a salt thereof, and squalene. The present invention also relates to the use of such a composition as a skin conditioning agent.

Skin is constantly exposed to the environment and its changing conditions, such as dryness, humidity, sunshine, UV radiation, coldness, and pollution. Furthermore, under normal living conditions, skin is exposed to a microbial flora, including bacterial and fungal germs and cells. In addition, skin may be exposed to mechanical stress.

In healthy skin, the outer layers of the skin are mechanically and physically protected by the *stratum corneum* and skin hair. Furthermore, various types of glands produce lipids that protect the skin from dehydration and detrimental microbes. For example, sebaceous glands produce sebum.

Skin secretions such as sebum have a number of functions, including preventing dehydration of the skin, lubricating the skin, balancing the hydrophobicity of the skin, providing oxidative resistance, maintaining and restoring elasticity and flexibility of the skin. Skin secretions may also play a role in thermoregulation or influence the microbial flora.

Skin homeostasis can be disturbed by external or internal factors. Such factors can detrimentally alter the composition of the sebaceous lipids (also skin lipids) contained in sebum as well as the moisture content of the skin. For example, environmental factors, climate, season changes, pollution, flights, wearing face masks, harsh surfactants, hyper hygiene, shaving, diet, stress, aging, hormones can lead to an imbalance in the composition of the sebaceous lipids or deprive the skin of skin lipids and skin moisture. This in turn can lead to dry skin, oily skin, sensitive skin, irritated skin, acne skin, for example.

There is an ongoing need for cosmetic products, in particular skin care products, and ingredients therefor, such as emollients. In particular, there is a need for ingredients, such as emollients, that protect the skin, help it to recover from the loss of natural lipids and keep it moist. Consumers have generally become more conscious about the ingredients used in cosmetic products. Among others, they desire sustainable and environmentally friendly ingredients that are derived from renewable sources. There is also a desire for ingredients, such as emollients, that provide appealing sensations (e.g. a pleasant feel and after-feel) and appealing textures. Ideally, such ingredients can replace silicones in cosmetic formulations, since silicones in cosmetic products have come under increased scrutiny.

It has now been found that a composition comprising a triglyceride-containing oil, jojoba oil, a free fatty acid or a salt thereof, and squalene provides various beneficial technical effects.

Accordingly, the invention relates to a composition comprising:
(A) 20 to 70% by weight of one or more oils comprising at least 75% by weight, referred to the total weight of the one or more oils, of one or more triglycerides as component A;
(B) 10 to 50% by weight of jojoba oil as component B;
(C) 5 to 30% by weight of one or more free fatty acids or salts thereof as component C;
(D) 5 to 25% by weight of squalene as component D; and
(E) 0 to 10% by weight of one or more antioxidants as component E.

The composition of the present invention is, for example, useful as a skin conditioning agent, such as an emollient. When applied to skin, it provides a pleasant (e.g. natural) feel as well as after-feel. It may replenish lost sebaceous lipids and/or may rebalance the composition of the sebaceous lipids. The composition of the present invention is convenient to use, and it shows a high stability to oxidation. The composition of the present invention can be derived from renewable sources.

Percentages by weight as used herein refer to the total weight of the respective component. In the context of the composition of the present invention, the percentages by weight (% by weight) refer to the total weight of the composition. In the context of a specific component, the percentages by weight (% by weight) refer to the total weight of the specific component.

Preferably, all components A to D and, if present, E are cosmetically and/or pharmaceutically acceptable. Particularly preferably, all components A to D and, if present, E are cosmetically acceptable.

The composition of the present invention comprises one or more oils comprising at least 75% by weight, referred to the total weight of the one or more oils, of one or more triglycerides as component A.

The one or more oils of component A may be any oils (other than jojoba oil) that comprise at least 75% by weight, referred to the total weight of the one or more oils, of one or more triglycerides.

Triglycerides are abundant in sebum. Oils such as olive oil or sunflower oil bring triglycerides to replenish the skin lipids. Sunflower oil renders products less susceptible to oxidation processes.

Preferably, the one or more oils of component A are of natural origin. In a preferred embodiment, at least 50% by weight, or at least 75% by weight, or at least 90% by weight, or even 100% by weight of the one or more oils of component A, referred to the total weight of component A, are of natural origin.

Preferably, the one or more oils of component A are of plant origin. In a preferred embodiment, at least 50% by weight, or at least 75% by weight, or at least 90% by weight, or even 100% by weight of the one or more oils of component A, referred to the total weight of component A, are of plant origin. Preferably, the one or more oils of component A are plant oils. Each plant oil may be a crude plant oil or a refined plant oil.

In a preferred embodiment, the one or more oils of component A comprise at least 80% by weight, or at least 85% by weight, or at least 90% by weight, or at least 95% by weight, referred to the total weight of component A, of one or more triglycerides.

The fatty acid residues in the triglycerides may be saturated or unsaturated, unbranched or branched.

In one embodiment, component A comprises one or more oils selected from the group consisting of olive oil, sunflower oil, grape seed oil, coconut oil, argan oil, corn oil, soybean oil, rice oil, pumpkin oil, sesame oil, walnut oil, apricot oil, macadamia oil, avocado oil, sweet almond oil, castor oil, peanut oil, rapeseed oil, and abyssinian oil.

In a preferred embodiment, component A comprises olive oil. In a preferred embodiment, component A comprises at least 30% by weight, or at least 40% by weight, or at least 50% by weight, or at least 60% by weight, or at least 70% by weight, or at least 75% by weight, or at least 80% by weight, or at least 85% by weight, or at least 90% by weight, or at least 95% by weight, referred to the total weight of component A, of olive oil.

Olive oil is an oil of natural origin, in particular plant origin. It is commercially available. As used herein, the terms "olive oil", "*Olea europaea* oil", "*Olea europaea* olive oil", "*Olea europaea* (olive) oil" may be understood interchangeably.

In a preferred embodiment, component A comprises sunflower oil. In a preferred embodiment, component A comprises from 1 to 20% by weight, or from 2 to 15% by weight, or from 3 to 10% by weight, or from 4 and 8% by weight, referred to the total weight of component A, of sunflower oil.

Sunflower oil is an oil of natural origin, in particular plant origin. It is commercially available. As used herein, the terms "sunflower oil", "common sunflower oil", "sunflower seed oil", "common sunflower seed oil", *"Helianthus annuus* oil", *Helianthus annuus* seed oil", *"Helianthus annuus* (sunflower) seed oil" may be understood interchangeably.

In a preferred embodiment, component A comprises olive oil and one or more oils selected from the group consisting of sunflower oil, grape seed oil, coconut oil, argan oil, corn oil, soybean oil, rice oil, pumpkin oil, sesame oil, walnut oil, apricot oil, macadamia oil, avocado oil, sweet almond oil, castor oil, peanut oil, rapeseed oil, and abyssinian oil. In a preferred embodiment, component A comprises olive oil and one or more oils selected from the group consisting of sunflower oil, grape seed oil, coconut oil, and argan oil. In a preferred embodiment, component A comprises olive oil and sunflower oil.

In a preferred embodiment, component A comprises or consists of:
(A-i) 80 to 100% by weight, referred to the total weight of component A, of olive oil,
(A-ii) 0 to 20% by weight, referred to the total weight of component A, of sunflower oil.

In a preferred embodiment, component A comprises or consists of:
(A-i) 90 to 100% by weight, referred to the total weight of component A, of olive oil,
(A-ii) 0 to 10% by weight, referred to the total weight of component A, of sunflower oil.

In a preferred embodiment, component A comprises or consists of:
(A-i) 90 to 97% by weight, referred to the total weight of component A, of olive oil,
(A-ii) 3 to 10% by weight, referred to the total weight of component A, of sunflower oil.

The composition of the present invention comprises from 20 to 70% by weight of component A, referred to the total weight of the composition. In a preferred embodiment, the composition of the present invention comprises from 30 to 60% by weight, or from 35 to 50% by weight, or from 40 to 50% by weight, or from 43 to 44% by weight, of component A, referred to the total weight of the composition.

The composition of the present invention comprises jojoba oil as component B.

Jojoba oil is an oil of natural origin, in particular plant origin. It can be obtained from the seeds of the jojoba plant. Jojoba oil is commercially available, for example as Golden Jojoba Oil.

Jojoba oil provides hydrophobicity to the skin and contributes to a light sensoriality left on the skin. Jojoba oil renders products less susceptible to oxidation processes.

As used herein, the terms "jojoba oil", *"Simmondsia chinensis* seed oil", "jojoba seed oil", *"Simmondsia chinensis* oil", "golden jojoba oil", "jojoba seed wax", *"Simmondsia chinensis* seed wax", *"Simmondsia chinensis* wax", "golden jojoba wax" and "jojoba wax" may be understood interchangeably.

Jojoba oil typically contains only low amounts of triglycerides of preferably not more than 25% by weight, or not more than 20% by weight, or not more than 15% by weight, or not more than 10% by weight, or not more than 5% by weight, referred to the total weight of the jojoba oil. Jojoba oil typically contains high amounts of wax esters, such as monoesters of long-chain fatty acids, of preferably at least 75% by weight, or at least 80% by weight, or at least 85% by weight, or at least 90% by weight, or at least 95% by weight, referred to the total weight of the jojoba oil. Jojoba oil is stable against oxidation.

The composition of the present invention comprises from 10 to 50% by weight of component B, referred to the total weight of the composition. In a preferred embodiment, the composition of the present invention comprises from 15 to 40% by weight, or from 20 to 30% by weight, or from 24 to 28% by weight, or for example 26% by weight, of component B, referred to the total weight of the composition.

The composition of the present invention comprises one or more free fatty acids or salts thereof as component C.

As used herein, a free fatty acid is any fatty acid comprising a free carboxyl group (COOH).

A salt of a fatty acid preferably is a cosmetically and/or pharmaceutically acceptable salt, in particular a cosmetically acceptable salt. Such salt may comprise a fatty acid anion and any cosmetically acceptable cation. For example, such cosmetically acceptable cation may be selected from the group consisting of alkali metals (e.g., sodium and/or potassium), alkaline earth metals (e.g., magnesium and/or calcium), aluminum, ammonium, and a combination of two or more thereof.

Free fatty acids or salts thereof are abundant in sebum. Free fatty acids or salts thereof increase skin permeability and facilitate and support the entry of nutrients and other ingredients into the skin.

Free fatty acids or salts thereof may be obtained from any source. Free fatty acids or salts thereof can be found directly in a natural source such as a plant-derived material or may be obtained by saponification of one or more oily components such as, e.g., triglycerides.

Preferably, the one or more free fatty acids or salts thereof are of natural origin. In a preferred embodiment, at least 50% by weight, or at least 75% by weight, or at least 90% by weight, or even 100% by weight of the one or more free fatty acids or salts thereof, referred to the total weight of the one or more free fatty acids or salts thereof, are of natural origin.

Preferably, the one or more free fatty acids or salts thereof are of plant origin. In a preferred embodiment, at least 50% by weight, or at least 75% by weight, or at least 90% by weight, or even 100% by weight of the one or more free fatty acids or salts thereof, referred to the total weight of the one or more free fatty acids or salts thereof, are of plant origin.

Preferably, the free fatty acids or salts thereof have 8 to 28 carbon atoms, more preferably 12 to 24 carbon atoms, even more preferably 14 to 22 carbon atoms, even more preferably 16 to 20 carbon atoms, particularly preferably 18 carbon atoms. The free fatty acids or salts thereof may be unbranched or branched. The free fatty acids or salts thereof may be saturated or unsaturated. In this context, "unsaturated" means that the free fatty acids or salts thereof have one, two, three or more double bonds in the fatty acid chain.

In one preferred embodiment, the free fatty acids or salts thereof are unbranched. In one preferred embodiment, the free fatty acids or salts thereof are unsaturated. In one preferred embodiment, the free fatty acids or salts thereof are monounsaturated, which means that they have one double bond in the fatty acid chain. In a more preferred embodiment, the free fatty acids or salts thereof are unbranched and unsaturated. In a particularly preferred embodiment, the free fatty acids or salts thereof are unbranched and monounsaturated.

Examples of fatty acids or salts thereof are oleic acid, caprylic acid, capric acid, lauric acid, myristic acid, palmitic acid, stearic acid, arachidic acid, behenic acid, lignoceric acid, cerotic acid, myristoleic acid, palmitoleic acid, sapienic acid, elaidic acid, vaccenic acid, linoleic acid, linoelaidic acid, linolenic acid, arachidonic acid, eicosapentaenoic acid, erucic acid, docosahexaenoic acid, or salts thereof.

In a preferred embodiment, the one or more free fatty acids or salts thereof of component C comprise oleic acid or a salt thereof. In a particularly preferred embodiment, the one or more free fatty acids or salts thereof of component C comprise oleic acid.

In a preferred embodiment, the one or more free fatty acids or salts thereof of component C comprise at least 75% by weight, or at least 90% by weight, or at least 95% by weight, or at least 99% by weight, referred to the total weight of component C, of oleic acid or a salt thereof. In a particularly preferred embodiment, the one or more free fatty acids or salts thereof of component C (essentially) consist of oleic acid or a salt thereof.

The composition of the present invention comprises from 5 to 30% by weight of component C, referred to the total weight of the composition. In a preferred embodiment, the composition of the present invention comprises from 8 to 25% by weight, or from 10 to 20% by weight, or from 14 to 18% by weight, or for example 16% by weight, of component C, referred to the total weight of the composition.

The composition of the present invention comprises squalene as component D.

Squalene is present in skin secretion such as sebum. Squalene protects skin from UV radiation and from free radicals.

Squalene may be obtained from any source. Squalene may, for example, be of animal origin (e.g. shark liver oil) or plant origin (e.g. olive oil, amaranth seed oil, rice bran oil, wheat germ oil). Squalene may also be obtained by genetic engineering methods (e.g. using bacteria or yeast).

Preferably, squalene is of natural origin. In a preferred embodiment, at least 50% by weight, or at least 75% by weight, or at least 90% by weight, or even 100% by weight of component D, referred to the total weight of component D, are of natural origin.

Preferably, squalene is of plant origin. In a preferred embodiment, at least 50% by weight, or at least 75% by weight, or at least 90% by weight, or even 100% by weight of component D, referred to the total weight of component D, are of plant origin.

In a preferred embodiment, component D is phytosqualene. Phytosqualene refers to squalene of plant origin.

The composition of the present invention comprises from 5 to 25% by weight of component D, referred to the total weight of the composition. In a preferred embodiment, the composition of the present invention comprises from 8 to 20% by weight, or from 10 to 15% by weight, or from 10 to 14% by weight, or for example 12% by weight, of component D, referred to the total weight of the composition.

The composition of the present invention comprises one or more antioxidants as component E.

The one or more antioxidants may be any agents having antioxidant properties.

Antioxidants preserve products from oxidation and prolong their shelf life.

Antioxidants may be obtained from any source. Antioxidants may, for example, be of plant origin (e.g. plant oil), microbial origin (e.g. bacterial or fungal cultures) or synthetic origin. Antioxidants may also be obtained by genetic engineering methods (e.g. using bacteria or yeast).

Preferably, the one or more antioxidants are of natural origin. In a preferred embodiment, at least 50% by weight, or at least 75% by weight, or at least 90% by weight, or even 100% by weight of the one or more antioxidants, referred to the total weight of the one or more antioxidants, are of natural origin.

Preferably, the one or more antioxidants are of plant origin. In a preferred embodiment, at least 50% by weight, or at least 75% by weight, or at least 90% by weight, or even 100% by weight of the one or more antioxidants, referred to the total weight of the one or more antioxidants, are of plant origin.

Preferred antioxidants are vitamins or derivatives thereof, or naringenin. In this context, "derivatives" means amide, ether, ester, amino, carboxyl, acetyl, acid, salt or alcohol derivatives. Preferred vitamins are vitamin E or vitamin C. A particularly preferred antioxidant is tocopherol. In a particularly preferred embodiment, component E is tocopherol.

In a preferred embodiment, the one or more antioxidants of component E comprise tocopherol. In a particularly preferred embodiment, the one or more antioxidants of component E (essentially) consist of tocopherol.

In a preferred embodiment, the composition of the present invention comprises from 0 to 10% by weight, or from 0 to 5% by weight, or from 0.1 to 10% by weight, or from 0.5 to 5% by weight, or from 1 to 5% by weight, or from 1 to 4% by weight, or from 2 to 3% by weight, of component E, referred to the total weight of the composition.

The composition of the present invention comprises
(A) 20 to 70% by weight of component A;
(B) 10 to 50% by weight of component B;
(C) 5 to 30% by weight of component C;
(D) 5 to 25% by weight of component D; and
(E) 0 to 10% by weight, preferably 0.1 to 10% by weight, of component E.

In a preferred embodiment, the composition comprises or consists of:
(A) 30 to 60% by weight of component A;
(B) 15 to 40% by weight of component B;
(C) 8 to 25% by weight of component C;
(D) 8 to 20% by weight of component D; and
(E) 0 to 5% by weight, preferably 0.5 to 5% by weight, of component E.

In a preferred embodiment, the composition comprises or consists of:
(A) 35 to 50% by weight of component A;
(B) 20 to 30% by weight of component B;
(C) 10 to 20% by weight of component C;
(D) 10 to 15% by weight of component D; and
(E) 0 to 5% by weight, preferably 1 to 5% by weight, of component E.

In a preferred embodiment, the composition comprises or consists of:
(A) 40 to 50% by weight of component A;
(B) 20 to 30% by weight of component B;
(C) 10 to 20% by weight of component C;
(D) 10 to 15% by weight of component D; and
(E) 1 to 4% by weight of component E.

In a preferred embodiment, the composition comprises or consists of:
(A) 35 to 50% by weight of one or more oils comprising at least 80% by weight, referred to the total weight of component A, of olive oil as component A;
(B) 20 to 30% by weight of jojoba oil as component B;
(C) 10 to 20% by weight of oleic acid as component C;
(D) 10 to 15% by weight of squalene as component D; and
(E) 0 to 5% by weight, preferably 1 to 5% by weight, of tocopherol as component E.

In a preferred embodiment, the composition comprises or consists of:
(A) 40 to 50% by weight of one or more oils comprising at least 90% by weight, referred to the total weight of component A, of olive oil as component A;
(B) 20 to 30% by weight of jojoba oil as component B;
(C) 10 to 20% by weight of oleic acid as component C;
(D) 10 to 15% by weight of squalene as component D; and
(E) 1 to 4% by weight of tocopherol as component E.

In a preferred embodiment, the composition comprises or consists of:
(A) 43 to 44% by weight of one or more oils comprising at least 90% by weight, referred to the total weight of component A, of olive oil as component A;
(B) 26% by weight of jojoba oil as component B;
(C) 16% by weight of oleic acid as component C;
(D) 12% by weight of squalene as component D; and
(E) 2 to 3% by weight of tocopherol as component E.

Preferably, the composition of the present invention has a renewable carbon index (RCI) of at least 75%, more preferably at least 80%, even more preferably at least 85%, even more preferably at least 90%, particularly preferably at least 95%. The renewable carbon index (RCI) as used herein is determined according to ISO 16128. The reference number of ISO 16128 as referred to herein is ISO 16128-1:2016(E).

In a preferred embodiment, at least 50% by weight, or at least 75% by weight, or at least 90% by weight, or even 100% by weight of the composition of the present invention, referred to the total weight of said composition, is of natural origin.

In a preferred embodiment, at least 50% by weight, or at least 75% by weight, or at least 90% by weight, or even 100% by weight of the composition of the present invention, referred to the total weight of said composition, is of plant origin.

In a preferred embodiment, at least components A, B and C, and optionally component D, and optionally component E, if present, are of plant origin. In a preferred embodiment, at least components A, B, C and D, and optionally component E, if present, are of plant origin. In a preferred embodiment, components A, C and D are obtained from olive, in particular from olive oil. In a preferred embodiment, at least components A, B, C and D are obtained from olive or jojoba. In a preferred embodiment, the composition of the present invention is free of palm oil. In a preferred embodiment, the composition of the present invention is free of palm oil and coconut oil.

Components of natural origin are preferred over components of synthetic origin. For example, components of natural origin have a better ecological footprint. They can be obtained from renewable sources. Their use is therefore more sustainable.

Components of plant origin are preferred over components of animal origin. This may not only be for ethical reasons but also for technical reasons, for example to avoid health risks arising from the potential transmission of diseases.

The composition of the present invention may be prepared by methods known in the art. For example, the composition may be prepared by mixing its ingredients, preferably at ambient temperature (20°C) and ambient pressure (1013 mbar).

Advantageously, the composition of the present invention is stable, in particular storage-stable. In preferred embodiments, the composition of the present invention is liquid at ambient temperature (20°C) and ambient pressure (1013 mbar). In preferred embodiments, the composition is cold-processable.

The composition of the present invention may, for example, be used as an ingredient in a formulation such as a cosmetic formulation or a pharmaceutical formulation. Such a formulation may further comprise one or more cosmetically and/or pharmaceutically acceptable ingredients. In a preferred embodiment, the composition of the present invention is used as an ingredient in a cosmetic formulation. Preferably, such a formulation further comprises one or more cosmetically acceptable ingredients.

Accordingly, the invention also relates to a formulation comprising:
(I) 0.1 to 50% by weight of a composition as described herein; and
(II) 50 to 99.9% by weight of one or more cosmetically acceptable ingredients.

The embodiments and examples described herein for the composition of the present invention apply also to the composition comprised in the formulation of the present invention.

In a preferred embodiment, the formulation comprises or consists of:
(I) 1 to 40% by weight of a composition as described herein; and
(II) 60 to 99% by weight of one or more cosmetically acceptable ingredients.

In a preferred embodiment, the formulation comprises or consists of:
(I) 3 to 30% by weight of a composition as described herein; and
(II) 70 to 97% by weight of one or more cosmetically acceptable ingredients.

In a preferred embodiment, the formulation comprises or consists of:
(I) 5 to 25% by weight of a composition as described herein; and
(II) 75 to 95% by weight of one or more cosmetically acceptable ingredients.

In a preferred embodiment, the formulation comprises or consists of:
(I) 7 to 20% by weight of a composition as described herein; and
(II) 80 to 93% by weight of one or more cosmetically acceptable ingredients.

In a particularly preferred embodiment, the formulation of the present invention is a cosmetic formulation. In another preferred embodiment, the formulation of the present invention may be a pharmaceutical formulation. In another preferred embodiment, the formulation of the present invention may be a dermatological formulation.

In a preferred embodiment, the formulation is a cosmetic formulation selected from the group consisting of skin care oil, skin care cream, skin care lotion, ointment, skin conditioner, face spray, body spray, hand oil, shower bath, hair conditioner, shaving gel, shampoo, body wash, facial cleanser, face mask, bubble bath, intimate wash, bath oil, cleansing milk, micellar water, make-up remover, cleansing wipes, hair mask, perfume, liquid soap, shaving soap, shaving stick, shaving foam, cleansing foam, day cream, anti-ageing cream, body milk, body lotion, body mousse, face serum, eye cream, sunscreen lotion, sun cream, face cream, after-shave lotion, pre-shaving cream, depilatory cream, skin-whitening gel, self-tanning cream, anti-acne gel, mascara, foundation, primer, concealer, blush, bronzer, blemish balm (bb) cream, eyeliner, night cream, eye brow gel, highlighter, lip stain, hand sanitizer, hair oil, nail varnish remover, conditioner, hair styling gel, hair styling cream, anti-frizz serum, scalp treatment, hair colorant, split end fluid, deodorant, antiperspirant, baby cream, insect repellent, hand cream, sunscreen gel, foot cream, exfoliator, body scrub, cellulite treatment, bar soap, cuticle cream, lip balm, hair treatment, eye shadow, bath additive, body mist, eau de toilette, mouthwash, toothpaste, lubricating gel, moisturizer, serum, toner, aqua sorbet, cream gel, styling mousse, dry shampoo, lip stick, lip gloss, body oil, shower milk, illuminator, lip crayon, hair spray, combing cream, and sunblock.

In a preferred embodiment, the formulation is a cosmetic formulation selected from the group consisting of skin care oil, skin care cream, skin care lotion, skin conditioner, face spray, body spray, hand oil, body wash, facial cleanser, face mask, cleansing milk, micellar water, liquid soap, cleansing foam, day cream, anti-ageing cream, body milk, body lotion, body mousse, face serum, eye cream, sunscreen lotion, sun cream, face cream, after-shave lotion, anti-acne gel, night cream, scalp treatment, baby cream, hand cream, sunscreen gel, foot cream, exfoliator, body scrub, cellulite treatment, moisturizer, body oil, shower milk, and sunblock.

In a preferred embodiment, the formulation is a skin care, hair care, and/or scalp care formulation. In a particularly preferred embodiment, the formulation is a skin care formulation. In a preferred embodiment, the formulation is a cosmetic formulation which is a skin care, hair care, and/or scalp care formulation. In a particularly preferred embodiment, the formulation is a cosmetic formulation which is a skin care formulation.

In at least one embodiment, the formulation comprises a solvent. In at least one embodiment, the formulation comprises a solvent, wherein the solvent comprises water and/or alcohol. Solvent is useful for providing the compounds used in present invention in liquid form. In at least one embodiment, the solvent is cosmetically acceptable. In at least one embodiment, the formulation comprises at least 10 wt.-%, preferably at least 20 wt.-%, more preferably at least 30 wt.-%, even more preferably at least 50 wt.-% water. Water is useful for economic reasons but also because it is cosmetically acceptable. Optionally, the formulation comprises water-miscible or water-soluble solvents, such as lower alkyl alcohols. In at least one embodiment, the formulation comprises C1-C5 alkyl monohydric alcohols, preferably C2-C3 alkyl monohydric alcohols. The alcohols which may be present are in particular lower monohydric or polyhydric alcohols having 1 to 4 carbon atoms customarily used for cosmetic purposes, such as preferably ethanol and isopropanol.

Optionally, the formulation comprises a water-soluble polyhydric alcohol. In at least one embodiment, the water-soluble polyhydric alcohols are polyhydric alcohols having two or more hydroxyl groups in the molecule. In at least one embodiment, the water-soluble polyhydric alcohol is selected from the group consisting of: dihydric alcohols such as ethylene glycol, propylene glycol, trimethylene glycol, 1,2-butylene glycol, 1,3-butylene glycol, 1,4-butylene glycol, tetramethylene glycol, 2,3-butylene glycol, pentamethylene glycol, 2-butene-1,4-diol, hexylene glycol, octylene glycol; trihydric alcohols such as glycerine, trimethylol propane, 1,2,6-hexanetriol and the like; tetrahydric alcohols such as pentaerythritol; pentahydric alcohols such as xylytol; hexahydric alcohols such as sorbitol, mannitol; polyhydric alcohol polymers such as diethylene glycol, dipropylene glycol, polyethylene glycol, polypropylene glycol, tetraethylene glycol, diglycerine, polyethylene glycol, triglycerine, tetraglycerine, polyglycerine; dihydric alcohol alkyl ethers such as ethylene glycol monomethyl ether, ethylene glycol monoethyl ether, ethylene glycol monobutyl ether, ethylene glycol monophenyl ether, ethylene glycol monohexyl ether, ethylene glycol mono-2-methylhexyl ether, ethylene glycol isoamyl ether, ethylene glycol benzyl ether, ethylene glycol isopropyl ether, ethylene glycol dimethyl ether, ethylene glycol diethyl ether, ethylene glycol dibutyl ether; dihydric alcohol alkyl ethers such as diethylene glycol monomethyl ether, diethylene glycol monoethyl ether, diethylene glycol monobutyl ether, diethylene glycol dimethyl ether, diethylene glycol diethyl ether, diethylene glycol butyl ether, diethylene glycol methyl ethyl ether, triethylene glycol monomethyl ether, triethylene glycol monoethyl ether, propylene glycol monomethyl ether, propylene glycol monoethyl ether, propylene glycol monobutyl ether, propylene glycol isopropyl ether, dipropylene glycol methyl ether, dipropylene glycol ethyl ether, dipropylene glycol butyl ether; dihydric alcohol ether esters such as ethylene glycol monomethyl ether acetate, ethylene glycol monoethyl ether acetate, ethylene glycol monobutyl ether acetate, ethylene glycol monophenyl ether acetate, ethylene glycol diadipate, ethylene glycol disuccinate, diethylene glycol monoethyl ether acetate, diethylene glycol monobutyl ether acetate, propylene glycol monomethyl ether acetate, propylene glycol monoethyl ether acetate, propylene glycol monopropyl ether acetate, propylene glycol monophenyl ether acetate; glycerine monoalkyl ethers such as xyl alcohol, selachyl alcohol, batyl alcohol; sugar alcohols such as sorbitol, maltitol, maltotriose, mannitol, sucrose, erythritol, glucose, fructose, starch sugar, maltose, xylose, starch sugar reduced alcohol, glysolid, tetrahydrofurfuryl alcohol, POE tetrahydrofurfuryl alcohol, POP butyl ether, POP POE butyl ether, tripolyoxypropylene glycerine ether, POP glycerine ether, POP glycerine ether phosphoric acid, POP POE pentaerythritol ether, and mixtures thereof.

In a preferred embodiment, the formulation comprises a solvent selected from the group consisting of water, glycols, ethanol, and mixtures thereof. In a preferred embodiment, the formulation comprises water. In one embodiment, the formulation is an aqueous solution.

In a preferred embodiment, the formulation comprises an aqueous, alcoholic or aqueous-alcoholic solvent, wherein the aqueous, alcoholic or aqueous-alcoholic solvent comprises water, ethanol, propanol, isopropanol, 1,2-propylene glycol, 1,3-propylene glycol, isobutanol, butanol, butyl glycol, butyl diglycol, glycerol, or mixtures thereof; preferably wherein the aqueous, alcoholic or aqueous-alcoholic solvent comprises water, ethanol, propanol, isopropanol, 1,2-propylene glycol, 1,3-propylene glycol, glycerol, or mixtures thereof; more preferably wherein the aqueous, alcoholic or aqueous-alcoholic solvent comprises water, isopropanol, 1,2-propylene glycol, 1,3-propylene glycol, or mixtures thereof; even more preferably wherein the aqueous, alcoholic or aqueous-alcoholic solvent consists of water or consists of a mixture of water and an alcohol wherein the alcohol is selected from the group consisting of isopropanol, 1,2-propylene glycol and 1,3-propylene glycol.

Natural solvents can also be used. In at least one embodiment, the formulation comprises a solvent selected from the group consisting of plant oil, honey, plant-derived sugar compositions, and mixtures thereof.

In at least one embodiment, the formulation comprises additives common in cosmetology, pharmacy, and dermatology, which are hereinafter called auxiliaries. In at least one embodiment, the auxiliary is selected from the group consisting of oily substances, emulsifiers, co-emulsifiers, cationic polymers, film formers, superfatting agents, stabilizers, active biogenic substances, glycerol, preservatives, pearlizing agents, dyes and fragrances, solvents, opacifiers, functional acids, and also protein derivatives such as gelatin, collagen hydrolysates, natural or synthetic-based polypeptides, egg yolk, lecithin, lanolin and lanolin derivatives, fatty alcohols, silicones, deodorants, substances with a keratolytic and keratoplastic action, enzymes, and/or carriers/solvents.

In at least one embodiment, the formulation comprises water soluble vitamins and their derivatives, water soluble amino acids and their salts and/or derivatives, viscosity modifiers, dyes, nonvolatile solvents or diluents (water soluble and insoluble), pearlescent aids, thickeners, foam boosters, surfactants or co-surfactants, pediculocides, pH adjusting agents, perfumes, preservatives, chelants, proteins, skin active agents, sunscreens, UV absorbers, vitamins, niacinamide, caffeine, minoxidil, and combinations thereof. In at least one embodiment, the formulation comprises from 0 wt.-% to 5 wt.-% vitamins and amino acids, by total weight of the formulation.

The formulation may also comprise pigment materials such as inorganic, nitroso, monoazo, diazo, carotenoid, triphenyl methane, triaryl methane, xanthene, quinoline, oxazine, azine, anthraquinone, indigoid, thionindigoid, quinacridone, phthalocyanine, botanical, natural colors, including: water soluble components such as those having C.I. Names. The formulation may comprise from 0 wt.-%, preferably from 0.0001 wt.-% to 5 wt.-% pigment materials.

In at least one embodiment, the formulation comprises an oily substance, which is any fatty substance which is liquid at room temperature (25°C). In at least one embodiment, the formulation comprises oily substance selected from the group consisting of silicone oils, volatile or nonvolatile, linear, branched or cyclic, optionally with organic modification; phenylsilicones; silicone resins and silicone gums; mineral oils such as paraffin oil or vaseline oil; oils of animal origin such as perhydrosqualene, lanolin; oils of plant origin such as liquid triglycerides, e.g., sunflower oil, corn oil, soybean oil, rice oil, jojoba oil, babusscu oil, pumpkin oil, grapeseed oil, sesame oil, walnut oil, apricot oil, macadamia oil, avocado oil, sweet almond oil, lady's-smock oil, castor oil, triglycerides of caprylic/capric acids, olive oil, peanut oil, rapeseed oil, argan oil, abyssinian oil, and coconut oil; synthetic oils such as purcellin oil, isoparaffins, linear and/or branched fatty alcohols and fatty acid esters, preferably guerbet alcohols having 6 to 18, preferably 8 to 10, carbon atoms; esters of linear (C₆-C₁₃) fatty acids with linear (C₆-C₂₀) fatty alcohols; esters of branched (C₆-C₁₃) carboxylic acids with linear (C₆-C₂₀) fatty alcohols, esters of linear (C₆-C₁₈) fatty acids with branched alcohols, especially 2-ethylhexanol; esters of linear and/or branched fatty acids with polyhydric alcohols (such as dimerdiol or trimerdiol, for example) and/or guerbet alcohols; triglycerides based on (C₆-C₁₀) fatty acids; esters such as dioctyl adipate, diisopropyl dimer dilinoleate; propylene glycols/dicaprylate or waxes such as beeswax, paraffin wax or microwaxes, alone or in combination with hydrophilic waxes, such as cetylstearyl alcohol, for example; fluorinated and perfluorinated oils; fluorinated silicone oils; mixtures of the aforementioned compounds.

In at least one embodiment, the formulation comprises a non-ionic co-emulsifier. In at least one embodiment, the non-ionic co-emulsifier is selected from adducts of from 0 to 30 mol of ethylene oxide and/or from 0 to 5 mol of propylene oxide with linear fatty alcohols having 8 to 22 carbon atoms, with fatty acids having 12 to 22 carbon atoms, with alkylphenols having 8 to 15 carbon atoms in the alkyl group, and with sorbitan or sorbitol esters; (C₁₂-C₁₈) fatty acid monoesters and diesters of adducts of from 0 to 30 mol of ethylene oxide with glycerol; glycerol monoesters and diesters and sorbitan monoesters and diesters of saturated and unsaturated fatty acids having 6 to 22 carbon atoms and, where appropriate, their ethylene oxide adducts; adducts of from 15 to 60 mol of ethylene oxide with castor oil and/or hydrogenated castor oil; polyol esters and especially polyglycerol esters, such as polyglyceryl polyricinoleate and polyglyceryl poly-12-hydroxystearate, for example. Likewise suitable are mixtures of compounds from one or more of these classes of substance. Examples of suitable ionogenic co-emulsifiers include anionic emulsifiers, such as mono-, di- or tri-phosphoric esters, but also cationic emulsifiers such as mono-, di-, or tri-alkyl quats and their polymeric derivatives.

In at least one embodiment, the formulation comprises a cationic polymer. Suitable cationic polymers include those known under the INCI designation "Polyquaternium", especially Polyquaternium-31, Polyquaternium-16, Polyquaternium-24, Polyquaternium-7, Polyquaternium-22, Polyquaternium-39, Polyquaternium-28, Polyquaternium-2, Polyquaternium-10, Polyquaternium-11, and also Polyquaternium 37 & mineral oil & PPG trideceth (Salcare SC95), PVP dimethylaminoethyl methacrylate copolymer, guar- hydroxypropyltriammonium chlorides, and also calcium alginate and ammonium alginate. It is additionally possible to employ cationic cellulose derivatives; cationic starch; copolymers of diallylammonium salts and acrylamides; quaternized vinylpyrrolidone/vinylimidazole polymers; condensation products of polyglycols and amines; quaternized collagen polypeptides; quaternized wheat polypeptides; polyethyleneimines; cationic silicone polymers, such as amidomethicones, for example; copolymers of adipic acid and dimethylaminohydroxypropyldiethylenetriamine; polyaminopolyamide and cationic chitin derivatives, such as chitosan, for example.

In at least one embodiment, the formulation comprises a superfatting agent. As superfatting agents it is possible to use substances such as, for example, polyethoxylated lanolin derivatives, lecithin derivatives, polyol fatty acid esters, monoglycerides, or fatty acid alkanol amides, the latter serving simultaneously as foam stabilizers. Moisturizers available include for example isopropyl palmitate, glycerol and/or sorbitol.

In at least one embodiment, the formulation comprises a stabilizer. As stabilizer it is possible to use metal salts of fatty acids, such as magnesium, aluminum and/or zinc stearate, for example.

In at least one embodiment, the formulation comprises a care additive. The formulations can be blended with conventional ceramides, pseudoceramides, fatty acid N-alkylpolyhydroxyalkyl amides, cholesterol, cholesterol fatty acid esters, fatty acids, triglycerides, cerebrosides, phospholipids, panthenol and similar substances as a care additive.

In at least one embodiment, the formulation comprises an anti-fungal substance. In at least one embodiment, the anti-fungal substance is selected from the group consisting of ketoconazole, oxiconazole, bifonazole, butoconazole, cloconazole, clotrimazole, econazole, enilconazole, fenticonazole, isoconazole, miconazole, sulconazole, tioconazole, fluconazole, itraconazole, terconazole, naftifine and terbinafine, zinc pyrithione, piroctone olamine (octopirox), and combinations thereof. In at least one embodiment, the formulation comprises a total amount of anti-fungal substance in the formulation of from 0.1 wt.-% to 1 wt.-%. In at least one embodiment, the formulation comprises pyridinethione anti-dandruff particulates. For example, 1-hydroxy-2-pyridinethione salts are highly preferred particulate anti-dandruff agents. The concentration of pyridinethione antidandruff particulate may range from 0.1 wt.-% to 4 wt.-%, by total weight of the formulation, preferably from 0.1 wt.-% to 3 wt.-%, more preferably from 0.3 wt.-% to 2 wt.-%. Preferred pyridinethione salts include those formed from heavy metals such as zinc, tin, cadmium, magnesium, aluminum or zirconium, preferably zinc, more preferably the zinc salt of 1-hydroxy-2-pyridinethione (known as "zinc pyridinethione" or "ZPT"), more preferably 1-hydroxy-2-pyridinethione salts in platelet particle form. Salts formed from other cations, such as sodium, may also be suitable.

Functional acids are acidic substances used to impart a clinical functionality to the skin or hair upon application. Suitable functional acids include alpha-hydroxy acids, beta-hydroxy acids, lactic acid, retinoic acid, and similar substances.

In at least one embodiment, the formulation comprises an astringent. In at least one embodiment, the astringent is selected from the group consisting of magnesium oxide, aluminum oxide, titanium dioxide, zirconium dioxide, zinc oxide, oxide hydrates, aluminum oxide hydrate (boehmite) and hydroxide, chlorohydrates of calcium, magnesium, aluminum, titanium, zirconium or zinc. In at least one embodiment, the formulation comprises from 0.001 wt.-% to 10 wt.-%, or from 0.01 wt.-% to 9 wt.-%, or from 0.05 wt.-% to 8 wt.-%, or from 0.1 wt.-% to 5 wt.-% astringent.

In at least one embodiment, the formulation comprises a deodorizing agent. In at least one embodiment, the deodorizing agent is selected from the group consisting of allantoin, bisabolol, and combinations thereof. In at least one embodiment, the formulation comprises from 0.001 wt.-% to 10 wt.-%, or from 0.01 wt.-% to 9 wt.-%, or from 0.05 wt.-% to 8 wt.-%, or from 0.1 wt.-% to 5 wt.-% deodorizing agent.

In at least one embodiment, the formulation comprises a sun protection agent and/or UV filter. Suitable sun protection agents and UV filters are disclosed in WO-2013/017262A1, from page 32, line 11 to the end of page 33. In at least one embodiment, the sun protection agent and/or UV filter is selected from the group consisting of 4-amino benzoic acid, 3-(4'-trimethylammonium)-benzylide-boran-2-one-methylsulfate, camphor benzalkonium methosulfate, 3,3,5-trimethyl-cyclohexylsalicylate, 2-hydroxy-4-methoxybenzophenone, 2-phenylbenzimidazole-5-sulfonic acid and potassium-, sodium- und triethanolamine salts thereof, 3,3'-(1,4-phenylene dimethine)-bis-(7,7-dimethyl-2-oxobicyclo[2.2.1]-heptane-1-methane sulfonic acid) and its salts, 1-(4-tert.-butylphenyl)-3-(4-methoxyphenyl)propan-1,3-dion, 3-(4'-sulfo)-benzylidene-bornane-2-one its salts, 2-cyan-3,3-diphenylacrylic acid-(2-ethylhexylester), polymers of N-[2(and 4)-(2-oxoborn-3-ylidenmethyl)benzyl]-acrylamide, 4-methoxy-cinnamic acid-2-ethyl-hexylester, ethoxylated ethyl-4-amino-benzoate, 4-methoxy-cinnamic acid-isoamylester, 2,4,6-tris-[p-(2-ethylhexyloxycarbonyl)anilino]-1,3,5-triazine, 2-(2H-benzotriazole-2-yl)-4-methyl-6-(2-methyl-3-(1,3,3,3-tetramethyl-1-(trimethylsilyloxy)-disiloxanyl)-propyl)phenol, 4,4'-[(6-[4-((1,1-dimethylethyl)-amino-carbonyl)phenylamino]-1,3,5-triazin-2,4-yl)diimino]bis-(benzoic acid-2-ethylhexylester), 3-benzophenone, 4-benzophenone (acid), 3(4'-methylbenzyliden)-D,L-camphor, 3-benzylidene-camphor, salicylic acid-2-ethylhexylester, 4-dimethyl aminobenzic acid-2-ethylhexylester, hydroxy-4-methoxy-benzophenone-5 sulfonic acid and the sodium salt thereof, 4-isopropyl benzylsalicylate, N,N,N-trimethyl-4-(2-oxoborn-3-ylidenemethyl) anilium methyl sulfate, homosalate (INN), oxybenzone (INN), 2-phenylbenzimidazole-5-sulfonic acid and its sodium, potassium, and triethanolamine salts, octylmethoxy cinnamic acid, isopentyl-4-methoxy cinnamic acid, isoamyl-p-methoxy cinnamic acid, 2,4,6-trianilino-(p-carbo-2'-ethylhexyl-1'-oxy)-1,3,5-triazine (octyl triazone) phenol, 2,2(2H-benzotriazol-2-yl)-4-methyl-6-(2-methyl-3-(1,3,3,3-tetramethyl-1-(trimethylsilyl)oxy)-disiloxanyl)propyl (drometrizole trisiloxane) benzic acid, 4,4-((6-(((1,1-dimethylethyl)amino)carbonyl)phenyl)amino)-1,3,5-triazine-2,4-diyl)diimino)bis,bis(2-ethylhexyl)ester) benzoic acid, 4,4-((6-(((1,1-dimethylethyl)amino)-carbonyl)phenyl)amino)-1,3,5-triazine-2,4-diyl)diimino)bis,bis(2-ethylhexyl)ester), 3-(4'-methylbenzylidene)-D,L-camphor (4-methylbenzylidene camphor), benzylidene-camphor-sulfonic acid, octocrylene, polyacrylamidomethyl-benzylidene-camphor, 2-ethylhexyl salicylate (octyl salicylate), 4-dimethyl-aminobenzoeacidethyl-2-hexylester (octyl dimethyl PABA), PEG-25 PABA, 2 hydroxy-4-methoxybenzo-phenone-5-sulfonic acid (5-benzophenone) and the sodium salt thereof, 2,2'-methylene-bis-6-(2H-benzotriazol-2-yl)-4-(tetramethyl-butyl)-1,1,3,3-phenol, the sodium salt of 2-2'-bis-(1,4-phenylene)1H-benzimidazole-4,6-disulfonic acid, (1,3,5)-triazine-2,4-bis((4-(2-ethyl-hexyloxy)-2-hydroxy)-phenyl)-6-(4-methoxyphenyl), 2-ethylhexyl-2-cyano-3,3-diphenyl-2-propenoate, glyceryl octanoate, di-p-methoxy cinnamic acid, p-aminobenzoic acid and its ester, 4-tert-butyl-4'-methoxydibenzoylmethane, 4-(2-β-glucopyranoxy)propoxy-2-hydroxybenzophenone, octyl salicylate, methyl-2,5-diisopropyl cinnamic acid, cinoxate, dihydroxy-dimethoxybenzophenone, disodium salts of 2,2'-dihydroxy-4,4'-dimethoxy-5,5'-disulfobenzophenone, dihydroxybenzophenone, 1,3,4-dimethoxyphenyl-4,4-dimethyl-1,3-pentanedione, 2-ethylhexyl-dimethoxybenzyliden-dioxoimidazolidinpropionate, methylene-bis-benztriazolyl tetramethylbutylphenol, phenyldibenzimidazoltetrasulfonate, bis-ethylhexyloxyphenol-methoxyphenol-triazine, tetrahydroxybenzophenone, terephthalylidendicamphor-sulfonic acid, 2,4,6-tris[4,2-ethylhexyloxycarbonyl)anilino]-1,3,5-triazine, methyl-bis(trimethylsiloxy)silyl-isopentyl trimethoxy cinnamic acid, amyl-p-dimethylaminobenzoate, amyl-p-dimethylamino benzoate, 2-ethylhexyl-p-dimethylaminobenzoate, isopropyl-p-methoxy cinnamic acid/diisopropyl cinnamic acid ester, 2-ethylhexyl-p-methoxy cinnamic acid, 2-hydroxy-4-methoxy benzophenone, 2-hydroxy-4-methoxybenzophenone-5-sulfonic acid and the trihydrate, 2-hydroxy-4-methoxybenzophenone-5-sulfonate sodium salt, phenyl-benzimidazole sulfonic acid, and combinations thereof. In at least one embodiment, the formulation comprises from 0.001 wt.-% to 10 wt.-%, preferably from 0.05 wt.-% to 5 wt.-%, even more preferably from 0.1 wt.-% to 3 wt.-%, most preferably from 0.05 wt.-% to 1 wt.-% sun protection agent and/or UV filter. In at least one embodiment, the formulation comprises a photoprotective substance in an amount of from 0.01 to 10 wt.-%, or from 0.1 to 5 wt.-%, more preferably from 0.2 to 2 wt.-%. Suitable photoprotective substances include, in particular, all of the photoprotective substances specified in EP1084696A1, which is incorporated herein by reference. In a preferred embodiment, the photoprotective substance is selected from the group consisting of 2-ethylhexyl 4-methoxycinnamate, methyl methoxycinnamate, 2-hydroxy-4-methoxybenzophenone-5-sulfonic acid, polyethoxylated p-aminobenzoates, and combinations thereof.

In at least one embodiment, the formulation comprises an anti-oxidant. In at least one embodiment, the anti-oxidant is selected from the group consisting of amino acids, peptides, sugars, imidazoles, carotinoids, carotenes, chlorogenic acid, lipoic acid, thiols, thiol glycosyl esters, thiol N-acetyl esters, thiol methyl esters, thiol ethyl esters, thiol propyl esters, thiol amyl esters, thiol butyl esters, thiol lauryl esters, thiol palmitoyl esters, thiol oleyl esters, thiol linoleyl esters, thiol cholesteryl esters, thiol glyceryl esters, dilaurylthiodipropionate, distearylthiodipropionate, thiodipropionic acid, metal chelators, hydroxy acids, fatty acids, folic acids, vitamin C, tocopherol, vitamin A, stilbenes, derivatives and combinations thereof. In at least one embodiment, the anti-oxidant is selected from the group consisting of glycine, histidine, tyrosine, tryptophan, urocaninic acid, D,L-carnosine, D-carnosine, L-carnosine, beta-carotene, alpha-carotene, lycopene, dihydrolipoic acid, aurothioglucose, propylthiouracil, thioredoxine, glutathione, cysteine, cystine, cystamine, buthioninsulfoximine, homocysteinsulfoximine, buthioninsulfone, penta-, hexa-, heptathioninsulfoximine, hydroxyfatty acids, palmitic acid, phytinic acid, lactoferrin, citric acid, lactic acid, malic acid, humic acid, bile acid, bilirubin, biliverdin, EDTA, EGTA, linoleic acid, linolenic acid, oleic acid, butylhydroxyanisol, trihydroxybutyrophenone, ubichinon, ubichinol, ascorbylpalmitate, Mg-ascorbylphosphate, ascorbylacetate, vitamin E acetate, vitamin A palmitate, carnosine, mannose, ZnO, ZnSO4, selenium methionine, stilbenes, superoxide dismutase, and combinations thereof. In at least one embodiment, the antioxidant is selected from the group consisting of vitamin A, vitamin A derivatives, vitamin E, vitamin E derivatives, and combinations thereof. In at least one embodiment, the formulation comprises from 0.001 wt.-% to 10 wt.-%, preferably from 0.05 wt.-% to 5 wt.-%, particularly preferably from 0.1 wt.-% to 3 wt.-%, also particularly preferably from 0.05 wt.-% to 1 wt.-% antioxidant.

In at least one embodiment, the formulation comprises a dye or pigment. In at least one embodiment, the formulation comprises at least one pigment. Suitable dyes and pigments are disclosed in WO2013/017262A1 in the table spanning pages 36 to 43. These may be colored pigments which impart color effects to the product mass or to hair, or they may be luster effect pigments which impart luster effects to the product mass or to hair. The color or luster effects on hair are preferably temporary, i.e. they last until the next hair wash and can be removed again by washing the hair with customary shampoos. In at least one embodiment, the formulation comprises a total amount of from 0.01 wt.-% to 25 wt.-%, preferably from 5 wt.-% to 15 wt.-% pigment. In at least one embodiment, the particle size of the pigment is from 1 micron to 200 micron, preferably from 3 micron to 150 micron, more preferably from 10 micron to 100 micron. The pigments are colorants which are virtually insoluble in the application medium, and may be inorganic or organic. Inorganic-organic mixed pigments are also possible. Preference is given to inorganic pigments. The advantage of inorganic pigments is their excellent resistance to light, weather and temperature. The inorganic pigments may be of natural origin. In at least one embodiment, the inorganic pigment is selected from the group consisting of chalk, ochre, umber, green earth, burnt sienna, graphite, and combinations thereof. The pigments may be white pigments, such as, for example, titanium dioxide or zinc oxide, black pigments, such as, for example, iron oxide black, colored pigments, such as, for example, ultramarine or iron oxide red, luster pigments, metal effect pigments, pearlescent pigments, and fluorescent or phosphorescent pigments, where preferably at least one pigment is a colored, nonwhite pigment. In at least one embodiment, the pigment is selected from the group consisting of metal oxides, hydroxides and oxide hydrates, mixed phase pigments, sulfur-containing silicates, metal sulfides, complex metal cyanides, metal sulfates, chromates and molybdates, and the metals themselves (bronze pigments), and combinations thereof. In at least one embodiment, the pigment is selected from the group consisting of titanium dioxide (CI 77891), black iron oxide (CI 77499), yellow iron oxide (CI 77492), red and brown iron oxide (CI 77491), manganese violet (CI 77742), ultramarine (sodium aluminum sulfosilicates, CI 77007, Pigment Blue 29), chromium oxide hydrate (CI 77289), Prussian blue (ferric ferrocyanide, CI 77510), carmine (cochineal), and combinations thereof. In at least one embodiment, the pigment is selected from the group consisting of pearlescent and colored pigments based on mica which are coated with a metal oxide or a metal oxychloride, such as titanium dioxide or bismuth oxychloride, and optionally further color-imparting substances, such as iron oxides, Prussian blue, ultramarine, carmine etc. and where the color can be determined by varying the layer thickness. Such pigments are sold, for example, under the trade names Rona^{®}, Colorona^{®}, Dichrona^{®} and Timiron^{®} by Merck. In at least one embodiment, the pigment is selected from the group consisting of organic pigments such as sepia, gamboge, bone charcoal, Cassel brown, indigo, chlorophyll and other plant pigments. In at least one embodiment, the pigment is selected from the group consisting of synthetic organic pigments such as azo pigments, anthraquinoids, indigoids, dioxazine, quinacridone, phthalocyanine, isoindolinone, perylene and perinone, metal complex, alkali blue and diketopyrrolopyrrole pigments.

In at least one embodiment, the formulation comprises from 0.01 wt.-% to 10 wt.-%, preferably from 0.05 wt.-% to 5 wt.-%, of at least one particulate substance. Suitable substances are, for example, substances which are solid at room temperature (25°C) and are in the form of particles. In at least one embodiment, the particulate substance is selected from the group consisting of silica, silicates, aluminates, clay earths, mica, insoluble salts, in particular insoluble inorganic metal salts, metal oxides, e.g. titanium dioxide, minerals and insoluble polymer particles are suitable. The particles may be present in the formulation in undissolved, preferably stably dispersed form, and, following application to the keratin substrate and evaporation of the solvent, can deposit on the substrate in solid form. A stable dispersion can be achieved by providing the formulation with a yield point which is large enough to prevent the solid particles from sinking. An adequate yield point can be established using suitable gel formers in a suitable amount. In at least one embodiment, the particulate substance is selected from the group consisting of silica (silica gel, silicon dioxide) and metal salts, in particular inorganic metal salts, where silica is particularly preferred. Metal salts are, for example, alkali metal or alkaline earth metal halides, such as sodium chloride or potassium chloride; alkali metal or alkaline earth metal sulfates, such as sodium sulfate or magnesium sulfate.

In at least one embodiment, the formulation comprises a direct dye. Preferred among the direct dyes are the following compounds, alone or in combination with one another: Hydroxyethyl-2-nitro-p-toluidine, 2-hydroxyethylpicramic acid, 4-nitrophenylaminourea, tri(4-amino-3-methylphenyl)carbenium chloride (Basic Violet 2), 1,4-di-amino-9,10-anthracenedione (Disperse Violet 1), 1-(2-hydroxyethyl)amino-2-nitro-4-[di(2-hydroxyethyl)amino]benzene (HC Blue No. 2), 4-[ethyl-(2-hydroxyethyl)amino]-1-[(2-hydroxyethyl)amino]-2-nitrobenzene hydrochloride (HC Blue No. 12), 1-amino-4-[di(2-hydroxyethyl)amino]-2-nitrobenzene hydrochloride (HC Red No. 13), 4-amino-1-[(2-hydroxyethyl)amino]-2-nitrobenzene (HC Red No. 3), 4-amino-3-nitrophenol, 4-[(2-hydroxyethyl)amino]-3-nitrophenol, 1-amino-5-chloro-4-[(2,3-dihydroxypropyl)amino]-2-nitrobenzene (HC Red No. 10), 5-chloro-1,4-[di(2,3-dihydroxypropyl)amino]-2-nitrobenzene (HC Red No. 11), 2-chloro-6-ethylamino-4-nitrophenol, 2-amino-6-chloro-4-nitrophenol, 4-[(2-hydroxyethyl)amino]-3-nitro-1-trifluoromethylbenzene (HC Yellow No. 13), 8-amino-2-bromo-5-hydroxy-4-imino-6-{[3-(trimethylammonio)-phenyl]amino}-1(4H)-naphthalenone chloride (C.I. 56059; Basic Blue No. 99), 1-[(4-aminophenyl)azo]-7-(trimethylammonio)-2-naphthol chloride (C.I. 12250; Basic Brown No. 16), 1-[(4-amino-2-nitrophenyl)azo]-7-(trimethylammonio)-2-naphthol chloride (Basic Brown No. 17), 2-hydroxy-1-[(2-methoxyphenyl)azo]-7-(trimethylammonio)naphthalene chloride (C.I. 12245; Basic Red No. 76), 3-methyl-1-phenyl-4-{[3-(trimethylammonio)phenyl]azo}pyrazol-5-one chloride (C.I. 12719; Basic Yellow No. 57) and 2,6-diamino-3-[(pyridin-3-yl)azo]pyridine as well as the salts thereof. Particularly preferred among the aforesaid direct dyes are the following compounds, alone or in combination with one another: hydroxyethyl-2-nitro-p-toluidine, 2-hydroxyethylpicramic acid, 4-nitrophenylaminourea, tri(4-amino-3-methylphenyl)carbenium chloride (Basic Violet 2), 1,4-di-amino-9,10-anthracenedione (Disperse Violet 1), 1-(2-hydroxy-ethyl)amino-2-nitro-4-[di(2-hydro-xyethyl)amino]benzene (HC Blue No. 2), 4-[ethyl-(2-hydroxyethyl)amino]-1-[(2-hydroxyethyl)amino]-2-nitrobenzene hydrochloride (HC Blue No. 12), 1-amino-4-[di(2-hydroxyethyl)amino]-2-nitrobenzene hydrochloride (HC Red No. 13), 4-amino-1-[(2-hydroxyethyl)amino]-2-nitrobenzene (HC Red No. 3), 4-amino-3-nitrophenol, 4-[(2-hydroxyethyl)amino]-3-nitrophenol, 1-amino-5-chloro-4-[(2,3-dihydroxypropyl)amino]-2-nitrobenzene (HC Red No. 10), 5-chloro-1,4-[di(2,3-dihydroxypropyl)-amino]-2-nitrobenzene (HC Red No. 11), 2-chloro-6-ethylamino-4-nitrophenol, 2-amino-6-chloro-4-nitrophenol, 4-[(2-hydroxyethyl)amino]-3-nitro-1-trifluoromethylbenzene (HC Yellow No. 13), 8-amino-2-bromo-5-hydroxy-4-imino-6-{[3-(trimethylammonio)-phenyl]amino}-1(4H)-naphthalenone chloride (C.I. 56059; Basic Blue No. 99), 1-[(4-aminophenyl)azo]-7-(trimethylammonio)-2-naphthol chloride (C.I. 12250; Basic Brown No. 16), 1-[(4-amino-2-nitrophenyl)azo]-7-(trimethylammonio)-2-naphthol chloride (Basic Brown No. 17), 2-hydroxy-1-[(2-methoxyphenyl)azo]-7-(trimethylammonio)naphthalene chloride (C.I. 12245; Basic Red No. 76), 3-methyl-1-phenyl-4-{[3-(trimethylammonio)phenyl]azo}pyrazol-5-one chloride (C.I. 12719; Basic Yellow No. 57) and 2,6-diamino-3-[(pyridin-3-yl)azo]pyridine as well as the salts thereof. In at least one embodiment, the total quantity of direct dyes in the formulation amounts to 0.01 to 15 wt.-%, preferably 0.1 to 10 wt.-%, most preferred 0.5 to 8 wt.-%.

In at least one embodiment, the formulation comprises a conditioning agent. In at least one embodiment, the conditioning agent is a water insoluble, water dispersible, non-volatile, liquid that forms emulsified, liquid particles. In at least one embodiment, the conditioning agent is a silicone (e.g., silicone oil, cationic silicone, silicone gum, high refractive silicone, or silicone resin), an organic conditioning oil (e.g., hydrocarbon oils, polyolefins, or fatty esters), a cationic conditioning surfactant, a high melting point fatty compound, or combinations thereof.

In at least one embodiment, the conditioning agent is a silicone, and the formulation comprises from 0.01 % to 10 %, or from 0.1 % to 5 % silicone conditioning agent, by total weight of the formulation. Suitable silicone conditioning agents, and optional suspending agents for the silicone, are described in US-5,104,646. In at least one embodiment, the formulation comprises a silicone gum selected from the group consisting of polydimethylsiloxane, (polydimethylsiloxane) (methylvinylsiloxane) copolymer, poly(dimethylsiloxane) (diphenylsiloxane) (methylvinylsiloxane) copolymer, and mixtures thereof.

In at least one embodiment, the formulation comprises a terminal aminosilicone. "Terminal aminosilicone" as defined herein means silicone comprising one or more amino groups at one or both ends of the silicone backbone. In at least one embodiment, the formulation is substantially free of any silicone compound comprising pendant amino groups. In an embodiment, the formulation is substantially free of any silicone compound other than terminal aminosilicones. In at least one embodiment, the amino group at at least one terminus of the silicone backbone of the terminal aminosilicone is selected from the group consisting of primary amines, secondary amines and tertiary amines. In at least one embodiment, the formulation comprises a terminal aminosilicone conforming to Formula (S):

(R^{F})ₐG₃₋ₐ-Si-(-OSiG₂)ₙ-O-SiG₃-ₐ(R^{F})ₐ (S)

wherein
- G: is hydrogen, phenyl, hydroxy, or C₁-C₈ alkyl, preferably methyl;
- a: is an integer having a value from 1 to 3, preferably 1;
- n: is a number from 0 to 1,999;
- R^{F}: is a monovalent radical conforming to the general formula C_{q}H_{2q}L, wherein q is an integer having a value from 2 to 8 and L is selected from the following groups: -N(R^{T})CH₂-CH₂-N(R^{T})₂; -N(R^{T})₂; -N(R^{T})₃A⁻; -N(R^{T})CH₂-CH₂-NR^{T}H₂A⁻; wherein R^{T} is hydrogen, phenyl, benzyl, or a saturated hydrocarbon radical, preferably an alkyl radical having from 1 to 20 carbon atoms; A⁻ is a halide ion.

In at least one embodiment, the terminal aminosilicone corresponding to Formula (S) has a = 1, q = 3, G = methyl, n is from 1000 to 2500, alternatively from 1500 to 1700, and L is -N(CH₃)₂. A suitable terminal aminosilicone corresponding to Formula (S) has a = O, G = methyl, n is from 100 to 1500, or from 200 to 800, and L is selected from the following groups: -N(R^{T})CH₂-CH₂-N(R^{T})₂; -N(R^{T})₂; -N(R^{T})₃A⁻; -N(R^{T})CH₂-CH₂-NR^{T}H₂A⁻; wherein R^{T} is hydrogen, phenyl, benzyl, or a saturated hydrocarbon radical, preferably an alkyl radical from having from 1 to 20 carbon atoms; A⁻ is a halide ion, alternatively L is -NH₂. In at least one embodiment, the terminal aminosilicone is selected from the group consisting of bis-aminomethyl dimethicone, bis-aminoethyl dimethicone, bis-aminopropyl dimethicone, bis-aminobutyl dimethicone, and mixtures thereof. In an embodiment, the viscosity of the terminal aminosilicone is from 1,000 to 30,000 cPs, or from 5,000 to 20,000 cPs, measured at 25°C. In at least one embodiment, the formulation comprises from 0.1 % to 20 %, or from 0.5 % to 10 %, or from 1 % to 6 % terminal aminosilicone, by total weight of the formulation.

In at least one embodiment, the formulation comprises a high melting point fatty compound. The high melting point fatty compound has a melting point of 25°C or higher. In at least one embodiment, the high melting point fatty compound is selected from the group consisting of a fatty alcohol, fatty acid, fatty alcohol derivative, fatty acid derivative, and mixtures thereof. Non-limiting examples of the high melting point compounds are found in International Cosmetic Ingredient Dictionary, Fifth Edition, 1993, and CTFA Cosmetic Ingredient Handbook, Second Edition, 1992. The formulation may comprise from 0.1 % to 40 %, or from 1 % to 30 %, or from 1.5 % to 16 %, or from 1.5 % to 8 % of a high melting point fatty compound, by total weight of the formulation. This is advantageous in view of providing improved conditioning benefits such as slippery feel during the application to wet hair, softness and moisturized feel on dry hair. In at least one embodiment, the fatty alcohol is selected from the group consisting of: cetyl alcohol, stearyl alcohol, behenyl alcohol, and mixtures thereof. In at least one embodiment, the formulation comprises a linear fatty alcohol, wherein the linear fatty alcohol is comprised in a lamellar gel matrix. A lamellar gel matrix is suitable for providing various conditioning benefits such as slippery feel during the application to wet hair and softness and moisturized feel on dry hair. The linear fatty alcohol may comprise from 8 to 24 carbon atoms. In an embodiment, the linear fatty alcohol is selected from the group consisting of: cetyl alcohol, stearyl alcohol, and mixtures thereof. In an embodiment, the weight ratio of total linear fatty alcohol to terminal aminosilicone is from 0.5:1 to 10:1, or from 1:1 to 5:1, or from 2.4:1 to 2.7:1.

In at least one embodiment, the lamellar gel matrix comprises a cationic conditioning surfactant and a high melting point fatty compound. In view of providing the lamellar gel matrix, the cationic conditioning surfactant and the high melting point fatty compound are contained at a level such that the weight ratio of the cationic surfactant to the high melting point fatty compound is in the range of from 1:1 to 1:10, or from 1:1 to 1:6.

In at least one embodiment, the formulation comprises a cationic conditioning surfactant. In at least one embodiment, the formulation comprises from 0.05 % to 3.0 %, or from 0.075 % to 2.0 %, or from 0.1 % to 1.0 %, of cationic conditioning surfactant by total weight of the formulation. In at least one embodiment, the cationic conditioning surfactant is comprised in a lamellar gel matrix. In other words, the formulation comprises a lamellar gel matrix and the lamellar gel matrix comprises the cationic conditioning surfactant. In an embodiment, cationic conditioning surfactant is according to Formula (C): 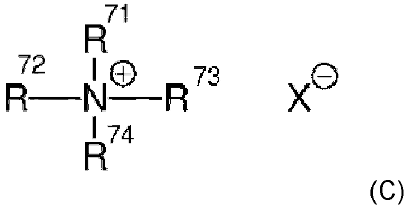
wherein at least one of R⁷¹, R⁷², R⁷³ and R⁷⁴ is selected from an aliphatic group of from 8 to 30 carbon atoms, an aromatic, alkoxy, polyoxyalkylene, alkylamido, hydroxyalkyl, aryl, or an alkylaryl group having up to 22 carbon atoms; the remainder of R⁷¹, R⁷², R⁷³ and R⁷⁴ are independently selected from the group consisting of an aliphatic group of from 1 to 22 carbon atoms, and an aromatic, alkoxy, polyoxyalkylene, alkylamido, hydroxyalkyl, aryl or alkylaryl group having up to 22 carbon atoms;
X is selected from the group consisting of: halogen, acetate, citrate, lactate, glycolate, phosphate, nitrate, sulfonate, sulfate, alkylsulfate, alkyl sulfonate, and combinations thereof.

In at least one embodiment, the cationic conditioning surfactant is selected from the group consisting of behenyl trimethyl ammonium chloride, methyl sulfate or ethyl sulfate, and stearyl trimethyl ammonium chloride, methyl sulfate or ethyl sulfate. It is believed that a longer alkyl group provides improved smoothness and soft feeling on wet and dry hair, compared to cationic surfactants with a shorter alkyl group. It is also believed that such cationic surfactants can provide reduced irritation, compared to those having a shorter alkyl group.

In at least one embodiment, the cationic surfactant is a di-long alkyl quaternized ammonium salt selected from the group consisting of: dialkyl (C14 - C18) dimethyl ammonium chloride, ditallow alkyl dimethyl ammonium chloride, dihydrogenated tallow alkyl dimethyl ammonium chloride, distearyl dimethyl ammonium chloride, dicetyl dimethyl ammonium chloride, and mixtures thereof.

In at least one embodiment, the cationic surfactant is a tertiary amido amine having an alkyl group of from 12 to 22 carbons. The tertiary amido amine may be selected from the group consisting of stearamidopropyldimethyl-, stearamidopropyldiethyl-, stearamidoethyldiethyl-, stearamidoethyldimethyl-, palmitamidopropyldimethyl-, palmitamidopropyldiethyl-, palmitamidoethyldiethyl-, palmitamidoethyldimethyl-, behenamidopropyldimethyl- behenamidopropyldiethyl-, behenamidoethyldiethyl-, behenamidoethyldimethyl-, arachidamidopropyldimethyl-, arachidamidopropyldiethyl-, arachidamidoethyldiethyl-, and arachidamidoethyldimethyl-amine, diethylaminoethylstearamide, and mixtures thereof. A tertiary amido amine may be used in combination with an acid. The acid is typically used as a salt-forming anion. In an embodiment, the acid is selected from the group consisting of lactic acid, malic acid, hydrochloric acid, 1-glumatic acid, acetic acid, citric acid, and mixtures thereof.

In at least one embodiment, the cationic surfactant is selected from the group consisting of cetyltrimethylammonium chloride (CTAC), stearyltrimethylammonium chloride (STAC), behentrimethylammonium methosulfate, stearoylamidopropyldimethyl amine (SAPDMA), distearyldimethylammonium chloride, and mixtures thereof.

In at least one embodiment, the formulation comprises a surfactant system. In at least one embodiment, the surfactant system comprises a surfactant selected from the group consisting of anionic surfactants, cationic surfactants, non-ionic surfactants, zwitterionic surfactants and/or amphoteric surfactants. In at least one embodiment, the formulation comprises a total amount of surfactant of from 0.01 wt.-% to 70 wt.-%, from 0.1 wt.-% to 40 %, from 1 wt.-% to 30 %, from 2 wt.-% to 20 wt.-%.

In at least one embodiment, the formulation comprises an anionic surfactant. In at least one embodiment, the anionic surfactant is selected from the group consisting of (C₁₀-C₂₀)-alkyl and alkylene carboxylates, alkyl ether carboxylates, fatty alcohol sulfates, fatty alcohol ether sulfates, alkylamide sulfates and sulfonates, fatty acid alkylamide polyglycol ether sulfates, alkanesulfonates and hydroxyalkanesulfonates, olefinsulfonates, acyl esters of isethionates, alpha-sulfo fatty acid esters, alkylbenzenesulfonates, alkylphenol glycol ether sulfonates, sulfosuccinates, sulfosuccinic monoesters and diesters, fatty alcohol ether phosphates, protein/fatty acid condensation products, alkyl monoglyceride sulfates and sulfonates, alkylglyceride ether sulfonates, fatty acid methyltaurides, fatty acid sarcosinates, sulforicinoleates, acylglutamates, and mixtures thereof. The anionic surfactants (and their mixtures) can be used in the form of their water-soluble or water-dispersible salts, examples being the sodium, potassium, magnesium, ammonium, mono, di-, and triethanolammonium, and analogous alkylammonium salts. In at least one embodiment, the anionic surfactant is the salt of an anionic surfactant comprising 12 to 14 carbon atoms. In at least one embodiment, the anionic surfactant is selected from the group consisting of sodium lauryl sulfate, sodium laureth sulfate, sodium tridecyl sulfate, sodium trideceth sulfate, sodium myristyl sulfate, sodium myreth sulfate, and mixtures thereof.

In at least one embodiment, the formulation comprises an acylglycinate surfactant. In at least one embodiment, the acylglycinate surfactant conforms to the formula (Y): 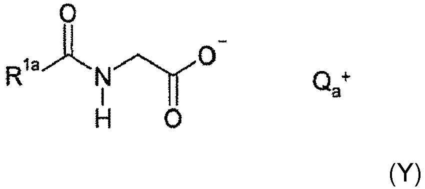 wherein
- R^{1a}: is a linear or branched, saturated alkyl group having 6 to 30, preferably 8 to 22, particularly preferably 8 to 18 carbon atoms or is a linear or branched, mono- or polyunsaturated alkenyl group having 6 to 30, preferably 8 to 22, particularly preferably 12 to 18 carbon atoms, and
- Qₐ⁺: is a cation.

In at least one embodiment, Qₐ⁺ is selected from the group consisting of Li⁺, Na⁺, K⁺, Mg⁺⁺, Ca⁺⁺, Al⁺⁺⁺, NH4⁺, a monoalkylammmonium ion, a dialkylammonium ion, a trialkylammonium ion and a tetraalkylammonium ion, or combinations thereof. In at least one embodiment, the acylglycinate surfactant is selected from sodium cocoylglycinate and potassium cocoylglycinate. In at least one embodiment, the acylglycinate surfactant is selected from those conforming to formula (Y), wherein R is C₁₂ alkyl or C₁₄ alkyl. In at least one embodiment, the acylglycinate surfactant is selected from those conforming to formula (Y), wherein R is C₁₆ alkyl or C₁₈ alkyl.

In at least one embodiment, the formulation comprises a glutamate surfactant corresponding to formula (Z) or a salt thereof: 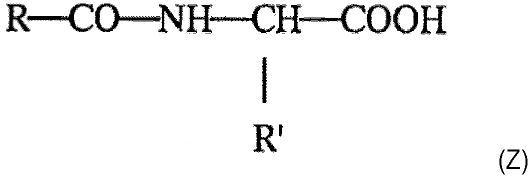 wherein
- R': is HOOC-CH2-CH2- or M⁺⁻OOC-CH2-CH2- wherein M⁺ is a cation;
- R: is a linear or branched, saturated alkyl group having 6 to 30, preferably 8 to 22, more preferably 8 to 18 carbon atoms or is a linear or branched, mono- or polyunsaturated alkenyl group having 6 to 30, preferably 8 to 22, more preferably 12 to 18 carbon atoms. In at least one embodiment, M⁺ is a metal cation. In at least one embodiment, M⁺ is selected from the group consisting of Li⁺, Na⁺, K⁺, Mg⁺⁺, Ca⁺⁺, Al⁺⁺⁺, NH4⁺, a monoalkylammmonium ion, a dialkylammonium ion, a trialkylammonium ion and a tetraalkylammonium ion, or combinations thereof.

In at least one embodiment, the glutamate surfactant is selected from sodium cocoyl glutamate and potassium cocoyl glutamate. In at least one embodiment, the glutamate surfactant is selected from those conforming to formula (Z), wherein R is C₁₂ alkyl or C₁₄ alkyl. In at least one embodiment, the glutamate surfactant is selected from those conforming to formula (Z), wherein R is C₁₆ alkyl or C₁₈ alkyl.

In at least one embodiment, the formulation comprises from 0.01 wt.-% to 30 wt.-%, preferably from 1 wt.-% to 25 wt.-%, more preferably from 5 wt.-% to 20 wt.-%, particularly preferably from 12 wt.-% to 18 wt.-% anionic surfactant.

In at least one embodiment, the formulation comprises a non-ionic surfactant. In at least one embodiment, the non-ionic surfactant has an HLB (Hydrophilic Lipophilic Balance) of greater than 12. Optionally, the non-ionic surfactant is selected from the group consisting of ethoxylated or ethoxylated/propoxylated fatty alcohols with a fatty chain having 12 to 22 carbon atoms, ethoxylated sterols, such as stearyl- or lauryl alcohol (EO-7), PEG-16 soya sterol or PEG-10 soya sterol, polyoxyethylene polyoxypropylene block polymers (poloxamers), and mixtures thereof.

In at least one embodiment, the non-ionic surfactant is selected from the group consisting of ethoxylated fatty alcohols, fatty acids, fatty acid glycerides or alkylphenols, in particular addition products of from 2 to 30 mol of ethylene oxide and/or 1 to 5 mol of propylene oxide onto C₈- to C₂₂-fatty alcohols, onto C₁₂- to C₂₂-fatty acids or onto alkyl phenols having 8 to 15 carbon atoms in the alkyl group, C₁₂- to C₂₂-fatty acid mono- and diesters of addition products of from 1 to 30 mol of ethylene oxide onto glycerol, addition products of from 5 to 60 mol of ethylene oxide onto castor oil or onto hydrogenated castor oil, fatty acid sugar esters, in particular esters of sucrose and one or two C₈- to C₂₂-fatty acids, INCI: Sucrose Cocoate, Sucrose Dilaurate, Sucrose Distearate, Sucrose Laurate, Sucrose Myristate, Sucrose Oleate, Sucrose Palmitate, Sucrose Ricinoleate, Sucrose Stearate, esters of sorbitan and one, two or three C₈- to C₂₂-fatty acids and a degree of ethoxylation of from 4 to 20, polyglyceryl fatty acid esters, in particular of one, two or more C₈- to C₂₂-fatty acids and polyglycerol having preferably 2 to 20 glyceryl units, alkyl glucosides, alkyl oligoglucosides and alkyl polyglucosides having C₈ to C₂₂-alkyl groups, e.g. decylglucoside or laurylglucoside, and mixtures thereof.

In at least one embodiment, the non-ionic surfactant is selected from the group consisting of fatty alcohol ethoxylates (alkylpolyethylene glycols), alkylphenol polyethylene glycols, alkylmercaptan polyethylene glycols, fatty amine ethoxylates (alkylaminopolyethylene glycols), fatty acid ethoxylates (acylpolyethylene glycols), polypropylene glycol ethoxylates (e.g. Pluronics^{®}), fatty acid alkylol amides (fatty acid amide polyethylene glycols), N-alkyl-, N-alkoxypolyhydroxy-fatty acid amides, sucrose esters, sorbitol esters, polyglycol ethers, and mixtures thereof.

In at least one embodiment, the formulation comprises a fatty N-methyl-N-glucamide surfactant, wherein the fatty N-methyl-N-glucamide surfactant conforms to the formula (X): 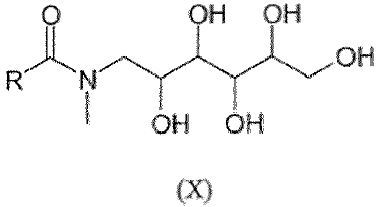 wherein R is selected from saturated or unsaturated hydrocarbon chains having 5 to 23 carbon atoms. Preferably, R in formula (X) is selected from saturated or unsaturated hydrocarbon chains having 7 to 17 carbon atoms. Also preferably, the R-C=O residue in formula (X) is derived from caprylic acid, capric acid, lauric acid, myristic acid, palmitic acid, stearic acid, oleic acid, linoleic acid, linolenic acid, coconut fatty acids, or mixtures thereof. Also preferably, the R-C=O residue in formula (X) is derived from 9-decenoic acid, 9-dodecenoic acid, or mixtures thereof.

Particularly preferred N-methyl-N-acylglucamines of formula (X) are capryloyl/caproyl methyl glucamide, lauroyl/myristoyl methyl glucamide, cocoyl methyl glucamide, oleyl methyl glucamide, or mixtures thereof. Such N-methyl-N-acylglucamines are commercially available from Clariant (GlucoTain^{®} Clear, GlucoTain^{®} Plus, GlucoTain^{®} Flex, GlucoTain^{®} Care, GlucoTain^{®} Sense).

Also particularly preferred N-methyl-N-acylglucamines of formula (X) are N-9-decenoyl-N-methylglucamine, N-9-dodecenoyl-N-methylglucamine, or mixtures thereof.

In at least one embodiment, the formulation comprises from 1 wt.-% to 20 wt.-%, preferably from 2 wt.-% to 10 wt.-%, more preferably from 3 wt.-% to 7 wt.-% non-ionic surfactant.

In at least one embodiment, the amphoteric surfactants are selected from the group consisting of N-(C₁₂-C₁₈)-alkyl-β-aminopropionates and N-(C₁₂-C₁₈)-alkyl-β-iminodipropionates as alkali metal salts and mono-, di-, and trialkylammonium salts; N-acylaminoalkyl-N,N-dimethylacetobetaine, preferably N-(C₈-C₁₈)-acylaminopropyl-N,N-dimethylacetobetaine, (C₁₂-C₁₈)-alkyl-dimethyl-sulfopropylbetaine, amphosurfactants based on imidazoline (e.g. Miranol^{®}, Steinapon^{®}), preferably the sodium salt of 1-(beta-carboxymethyloxyethyl)-1-(carboxymethyl)-2-laurylimidazolinium; amine oxides, e.g. (C₁₂-C₁₈)-alkyldimethylamine oxides, fatty acid amidoalkyldimethylamine oxides, and mixtures thereof.

In at least one embodiment, the formulation comprises a betaine surfactant. Optionally, the betaine surfactant is selected from C₈- to C₁₈-alkylbetaines. In at least one embodiment, the betaine surfactant is selected from the group consisting of cocodimethylcarboxymethylbetaine, lauryldimethylcarboxymethylbetaine, lauryldimethylalphacarboxyethylbetaine, cetyldimethylcarboxymethylbetaine, oleyldimethylgammacarboxypropylbetaine and laurylbis(2-hydroxypropyl)alphacarboxyethylbetaine, and combinations thereof. Optionally, the betaine surfactant is selected from C₈- to C₁₈-sulfobetaines. In at least one embodiment, the betaine surfactant is selected from the group consisting of cocodimethylsulfopropylbetaine, stearyldimethylsulfopropylbetaine, lauryldimethylsulfoethylbetaine, laurylbis(2-hydroxyethyl)sulfopropylbetaine, and combinations thereof. Optionally, the betaine surfactant is selected from carboxyl derivatives of imidazole, the C₈- to C₁₈-alkyldimethylammonium acetates, the C₈- to C₁₈-alkyldimethylcarbonylmethylammonium salts, and the C₈- to C₁₈-fatty acid alkylamidobetaines, and mixtures thereof. Optionally, the C₈- to C₁₈-fatty acid alkylamidobetaine is selected from coconut fatty acid amidopropylbetaine, N-coconut fatty acid amidoethyl-N-[2-(carboxymethoxy)ethyl]glycerol (CTFA name: Cocoamphocarboxyglycinate), and mixtures thereof.

In at least one embodiment, the formulation comprises from 0.5 wt.-% to 20 wt.-%, preferably from 1 wt.-% to 10 wt.-% amphoteric surfactant.

In at least one embodiment, the formulation comprises a surfactant system. In at least one embodiment, the surfactant system comprises at least one surfactant selected from the group consisting of lauryl sulfate, laureth sulfate, cocoamido-propylbetaine, sodium cocoylglutamate, lauroamphoacetate, and mixtures thereof. In at least one embodiment, the surfactant system comprises sodium laureth sulphate, sodium lauryl sulphate, and optionally cocamidopropyl betaine. In at least one embodiment, the surfactant system comprises sodium laureth sulphate, potassium cocoyl glutamate, and cocamidopropyl betaine.

In at least one embodiment, the formulation has a viscosity of from 0 cPs to 20,000 cPs. In at least one embodiment, the formulation has a viscosity of from 0.1 cPs to 10,000 cPs, or from 1 cPs to 5,000 cPs, or from 5 cPs to 3,500 cPs.

The viscosity measurement conditions are defined in the definitions section above. Viscosity may be important for anti-drip reasons. Dripping can be inconvenient for the user. Furthermore, more viscous formulations can be useful for measured dispensing. In at least one embodiment, the formulation has a viscosity of from 0 cPs to 1,000 cPs. This viscosity range is advantageous when the formulation is in the form of a facial cleanser in view of the need for distribution on skin and ability to rinse off.

In at least one embodiment, the formulation further comprises a viscosity-modifying substance. The viscosity-modifying substance is preferably a thickening polymer.

In at least one embodiment, the thickening polymer is a polymer based on acrylamidomethylpropanesulfonic acid (AMPS^{®}). These polymers, even at pH values of 7 or less, exhibit good thickening performance. Especially preferably, the thickening polymer is selected from the group consisting of homo- or copolymers of acrylamidomethylpropanesulfonic acid and salts thereof. Among the polymers just mentioned, preference is given to polymers having at least 20 mol-% of units based on acrylamidomethylpropanesulfonic acid and/or salts thereof, and particular preference to polymers having at least 50 mol-% of units based on acrylamidomethylpropanesulfonic acid and/or salts thereof, the mole figures relating in each case to the overall polymer. In the case of the copolymers, in addition to structural units based on acrylamidomethylpropanesulfonic acid and/or salts thereof, preferably one or more structural units based on the following comonomers are present in the copolymers: acrylic acid, methacrylic acid, acrylamide, dimethylacrylamide, vinylpyrrolidone (VP), hydroxyethyl acrylate, hydroxyethyl methacrylate, acrylic or methacrylic esters of ethoxylated alcohols RO-(CH₂CH₂O)ₘH, in which R is an alkyl radical having 12 to 30 carbon atoms and m is a number from 3 to 30, and CH₂=CH-COO-(CH₂CH₂-COO)ₙX, in which n is a number from 0 to 10 and X is a counterion and is preferably H⁺, Na⁺ and/or NH4⁺. The polymers selected from the group consisting of homo- or copolymers of acrylamidomethylpropanesulfonic acid and salts thereof may be crosslinked or non-crosslinked. In the case of crosslinking, they contain structural units based on monomers having 2 or more olefinic double bonds. In the case of crosslinking, preferably from 0.1 to 10 mol-% of such structural units are present in the homo- or copolymers, based on the overall polymer. If one or more structural units based on acrylamidomethylpropanesulfonic acid and/or salts thereof in the homo- or copolymers of acrylamidomethylpropanesulfonic acid and/or salts thereof have one or more counterions other than H⁺, these other counterions are preferably selected from the group consisting of Na⁺ and NH4⁺. Suitable polymers are mentioned in publications including EP-0816403, EP-1069142, EP-1116733 and DE-10 2009 014877 (Clariant), EP-1347736 (L'Oréal) or EP-1496081 (Seppic). Examples include: Aristoflex^{®} AVC (Ammonium Acryloyldimethyltaurate/VP Copolymer), Aristoflex^{®} AVS (Sodium Acryloyldimethyltaurate/VP Crosspolymer), Aristoflex^{®} TAC (Ammonium Acryloyl Dimethyltaurate Carboxyethyl Acrylate Crosspolymer), Hostacerin^{®} AMPS (Ammonium Polyacryloyldimethyl Taurate), Aristoflex^{®} HMB (Ammonium Acryloyldimethyltaurate/Beheneth-25 Methacrylate Crosspolymer), Aristoflex^{®} BLV (Ammonium Acryloyldimethyltaurate/Beheneth-25 Methacrylate Crosspolymer), Aristoflex^{®} HMS (Ammonium Acryloyldimethyltaurate/Steareth-25 Methacrylate Crosspolymer), Aristoflex^{®} SNC (Ammonium Acryloyldimethyltaurate/Steareth-8 Methacrylate Copolymer), Aristoflex^{®} LNC (Ammonium Acryloyldimethyltaurate/Laureth-7 Methacrylate Copolymer) or Sepinov^{®} EMT 10 (Hydroxyethyl Acrylate/Sodium Acryloyldimethyl Taurate Copolymer), Sepigel^{®} 305.

In at least one embodiment, the thickening polymer is selected from the group consisting of: copolymers of at least one first monomer type, which is chosen from acrylic acid and methacrylic acid, and at least one second monomer type, which is chosen from esters of acrylic acid and ethoxylated fatty alcohol; crosslinked polyacrylic acid; crosslinked copolymers of at least one first monomer type, which is chosen from acrylic acid and methacrylic acid, and at least one second monomer type, which is chosen from esters of acrylic acid with C₁₀- to C₃₀-alcohols; copolymers of at least one first monomer type, which is chosen from acrylic acid and methacrylic acid, and at least one second monomer type, which is chosen from esters of itaconic acid and ethoxylated fatty alcohol; copolymers of at least one first monomer type, which is chosen from acrylic acid and methacrylic acid, at least one second monomer type, which is chosen from esters of itaconic acid and ethoxylated C₁₀- to C₃₀-alcohols, and a third monomer type, which is chosen from C₁- to C₄-aminoalkyl acrylates; copolymers of two or more monomers chosen from acrylic acid, methacrylic acid, acrylic esters and methacrylic esters; copolymers of vinylpyrrolidone and ammonium acryloyldimethyltaurate; copolymers of ammonium acryloyldimethyltaurate and monomers chosen from esters of methacrylic acid and ethoxylated fatty alcohols; hydroxyethylcellulose; hydroxypropylcellulose; hydroxypropylguar; glyceryl polyacrylate; glyceryl polymethacrylate; copolymers of at least one C₂-, C₃- or C₄-alkylene and styrene; polyurethanes; hydroxypropyl starch phosphate; polyacrylamide; copolymers of maleic anhydride and methyl vinyl ether crosslinked with decadiene; carob seed flour; guar gum; xanthan; dehydroxanthan; carrageenan; karaya gum; hydrolyzed corn starch; copolymers of polyethylene oxide, fatty alcohols and saturated methylenediphenyl diisocyanate (e.g. PEG-150/stearyl alcohol/SMDI copolymer); and mixtures thereof.

In at least one embodiment, the formulation has a pH value of from 2.0 to 12.0, preferably from 3.0 to 9.0, more preferably from 4.5 to 7.5. By varying the pH value, a formulation can be made available that is suitable for different applications.

In at least one embodiment, the formulation comprises an alkalizing agent or pH adjusting agent. In at least one embodiment, ammonia or caustic soda is suitable, but water-soluble, physiologically tolerable salts of organic or inorganic bases can also be considered. Optionally, the pH adjusting agent is selected from ammonium hydrogen carbonate, ammonia, monoethanolamine, ammonium carbonate. In at least one embodiment, the alkalizing agent or pH adjusting agent is selected from the group consisting of 2-amino-2-methyl-1-propanol, 2-amino-2-methyl-1,3- propanediol, 2-amino-2-ethyl-1,3-propanediol, tris(hydroxymethyl)- aminomethane, 2-amino-1-butanol, tris(2-hydroxypropyl)-amine, 2,2-iminobisethanol, lysine, iminourea (guanidine carbonate), tetrahydro-1,4- oxazine, 2-amino-5-guanidin-valeric acid, 2-aminoethansulfonic acid, diethanolamine, triethanolamine, N-methyl ethanolamine, isopropanolamine, diisopropanolamine, triisopropanolamine, glucamine, sodium hydroxide, potassium hydroxide, lithium hydroxide, magnesium oxide, and mixtures thereof.

To establish an acidic pH value, an acid can be included. In at least one embodiment, the formulation comprises an acid selected from the group consisting of hydrochloric acid, phosphoric acid, acetic acid, formic acid, sulfuric acid, citric acid, and mixtures thereof. Citric acid is most preferred in that it has high consumer acceptance. In at least one embodiment, the acidic pH is adjusted with a buffer such as a phosphate buffer, a TRIS buffer or a citric buffer. The buffers may be used alone or in combination with an acid.

In at least one embodiment, the formulation is in liquid form. In an alternative embodiment, the formulation is in solid form. Optionally, the formulation is in powdered or granulated form. This is advantageous in that it is not needed to ship liquid, which is typically heavy, over long distances, which has economic and environmental benefits. A solid form can be achieved by spray drying the formulation or by using a rotary evaporator. The formulation can be converted into liquid form after it has been shipped, e.g. by adding water.

The formulation of the present invention may be prepared by methods known in the art. For example, the formulation may be prepared by mixing its ingredients, preferably at ambient temperature (20°C) and ambient pressure (1013 mbar).

The composition of the present invention or the formulation of the present invention may be used for any purpose. It may, for example, be used for cosmetic, pharmaceutical and/or dermatological purposes. In a preferred embodiment, the composition of the present invention or the formulation of the present invention is used for cosmetic purposes.

The formulation of the present invention may, for example, be used as a skin care, hair care and/or scalp care formulation. In a particularly preferred embodiment, the formulation is used as a skin care formulation.

The formulation of the present invention may, for example, be applied to skin, hair and/or scalp. Typically, the formulation is applied topically to skin, hair and/or scalp. In a particularly preferred embodiment, the formulation is applied to skin. Typically, the formulation is applied topically to skin. The formulation of the present invention may, for example, be a leave-on formulation or a rinse-off formulation.

The embodiments and examples described herein for the composition of the present invention or the formulation of the present invention apply also to the composition or the formulation used according to the present invention.

The composition used according to the present invention may be part of a formulation, preferably a formulation as described herein.

Preferably, the composition of the present invention is used as a skin conditioning agent. Accordingly, the invention also relates to the use of a composition as described herein as a skin conditioning agent. In preferred embodiments, the skin conditioning agent is an emollient, lubricant, moisturizer, refatting agent, smoothing agent and/or soothing agent. Particularly preferably, the skin conditioning agent is an emollient. Also preferably, the skin conditioning agent is a lubricant, moisturizer, refatting agent, smoothing agent and/or soothing agent.

Preferably, the composition of the present invention is used as an emollient, lubricant, moisturizer, refatting agent, smoothing agent and/or soothing agent. Accordingly, the invention also relates to the use of a composition as described herein as an emollient, lubricant, moisturizer, refatting agent, smoothing agent and/or soothing agent.

The uses as skin conditioning agents, emollients, lubricants, moisturizers, refatting agents, smoothing agents, soothing agents are cosmetic uses (not medical uses).

As used herein, the term "emollient" may be understood as generally understood in the field of cosmetics. An emollient may be an ingredient that makes skin and/or hair softer and smoother. An emollient may reduce skin roughness and make skin softer and smoother. Optionally, an emollient may concomitantly be a moisturizer.

The composition or formulation of the present invention may replenish lost sebaceous lipids and/or may rebalance the composition of the sebaceous lipids.

The following examples are intended to further illustrate the invention without limiting the scope thereof.

### Examples

### Example 1 - Example of a composition of the present invention

The blend was prepared by mixing its ingredients at room temperature. The ingredients listed in Table 1 were added one by one and stirred until the blend was homogeneous. The blend was obtained as a clear oily liquid.

**Table 1. Example of a composition of the present invention**

| **Ingredients** | **INCI name** | **Amount** |
|---|---|---|
| Plantasens^{®} Ref Olive Oil | Olea *europaea* Olive oil | 41% |
| Golden Jojoba Oil | *Simmondsia chinensis* seed oil | 26% |
| Oleic acid from Olive | Oleic acid | 16% |
| Plantasens^{®} Olive Squalene | Squalene | 12% |
| Plantasens^{®} Vitamin E Natural 50 | Tocopherol, *Helianthus annuus* (Sunflower) Seed oil | 5%* |

| | | |
|---|---|---|
| * 2.5% Tocopherol + 2.5% Helianthus annuus (Sunflower) Seed oil | | |

### Example 2 - Technical parameters and oxidative stability

The following technical parameters were determined for the blend of Example 1:
- Surface tension [mN/m], determined according to EN 14370:2004 [25 °C]: 32.5
- Spreadability [mm2/10 min]: 493
- Cloud point, determined according to DIN ISO 3016 [°C]: -3
- Refractive index, determined according to DIN ISO 6320 [20°C]: 1.4716

The spreadability was determined as follows:
- Vitro-Skin^{®} hydration protocol: Vitro-Skin^{®} pieces must be stored for a period of 16 to 24 hours on the shelf of a standardized hydration chamber with a 350 g mixture of glycerol (15%) and water (85%) at the bottom.
- The spreading value will be defined as the surface area covered by 10 µl of oil mixture after 10 minutes.
- The liquid will be dropped into the center of a hydrated Vitro-Skin^{®} piece measuring 65 x 65 mm using a manual micropipette from Fa. Mettler-Toledo LLC, Ohio, USA.
- The Vitro-Skin^{®} sample will be kept in the hydration chamber for 10 min according to the spreading phase.
- After 10 minutes, the outline of the spreading surface will be determined visually, and the spread will be determined using a ruler.
- The spreading area will be calculated using the circular area formula: *A* = *πr*² [A: area; π: circle number (3.14159...); r: radius of the circle calculated].
- Measurements will be performed in triplicate at room temperature.
- The spreading values are given in mm² (the higher the value, the higher the spread).

The oxidative stability at 90°C was determined according to DIN EN ISO 6886 for the blend of Example 1 and compared with the oxidative stability of squalene. The induction time for the blend of Example 1 (> 50 hours) was significantly higher than the induction time for squalene (4.84 hours). Accordingly, the blend of Example 1 is significantly more stable to oxidation than squalene. The composition of the present invention shows high stability to oxidation.

### Example 3 - Sensory evaluation

A sensory evaluation of the blend of Example 1 was conducted by a trained panel. The blend was applied to the skin. The panel found that the blend provides a pleasant feel during application as well as a pleasant after-feel. The composition of the present invention can be used as an emollient.

### Example 4 - Formulation example

An anti-stress replenishing face spray was prepared using the following ingredients:

| **Phase** | **Ingredient** | **%** |
|---|---|---|
| A | Aqua (water) | 86.8 |
| | Propanediol | 1.0 |
| | Aristoflex^{®} Silk (Sodium Polyacryloyldimethyl Taurate) | 0.4 |
| B | Hostaphat^{®} KL340 D (Trilaureth-4 Phosphate) | 2.0 |
| | Cetearyl Alcohol | 1.5 |
| | Blend of Example 1 | 7.0 |
| C | Velsan^{®} CGE (Caprylyl Glyceryl Ether) | 1.0 |
| | Parfum (Fragrance) | 0.3 |

This formulation targets, for example, skin with imbalanced sebaceous lipids.

### Example 5 - Formulation example

A regenerating booster hand oil was prepared using the following ingredients:

| **Phase** | **Ingredient** | **%** |
|---|---|---|
| A | Blend of Example 1 | 19.5 |
| | Caprylic/capric triglycerides | 10.0 |
| | Isopropyl Palmitate | 50.0 |
| | Cetearyl Isononanoate | 20.0 |
| B | Parfum (Fragrance) | 0.5 |

This formulation targets, for example, skin deprived of sebaceous lipids.

### Example 6 - Formulation example

A hydration shot face cream was prepared using the following ingredients:

| **Phase** | **Ingredient** | **%** |
|---|---|---|
| A | Water (Aqua) | 83.0 |
| | Glycerin | 2.0 |
| | Trisodium Citrate | 0.1 |
| | Aristoflex^{®} Silk (Sodium Polyacryloyldimethyl Taurate) | 1.0 |
| B | Blend of Example 1 | 10.0 |
| | Plantasens^{®} Emulsifier HP 30 (Glyceryl Stearate (and) Sodium Stearoyl Lactylate (and) Cetearyl Alcohol) | 2.0 |
| | Rainforest Bacuri Butter (Platonia Insignis Seed Butter) | 0.5 |
| C | Nipaguard^{®} SCE (Benzoic Acid (and) Sorbitan Caprylate) | 1.2 |
| | Fragrance (Golden Temptation) | 0.2 |

This formulation targets, for example, healthy skin with balanced sebaceous lipids.

### Example 7 - Rinse-off formulations

### Example 7a - Nourishing repair shampoo

| Phase | Ingredient | Function | |
|---|---|---|---|
| A | Water | | Ad 100% |
| | Carbopol^{®} Ultrez 21 (Acrylates / C10-30 Alkyl Acrylate Crosspolymer) | Rheology Modifier | 0.40% |
| B | Genapol^{®} LRO liq (Clariant) (Sodium Laureth Sulfate) | Surfactant | 37.00% |
| C | GlucoTain^{®} Care (Clariant) (Cocoyl Methyl Glucamide) | Co-Surfactant | 5.00% |
| | Blend of Example 1 | Emollient | 0.50% |
| | Fragrance AVOCADO (Cosnaderm) | Fragrance | 0.30% |
| D | Water | | 20.00% |
| | Jaguar^{®} C-162 (Hydroxypropyl Guar Hydroxypropyltrimonium Chloride) | Conditioning Agent | 0.10% |
| E | NaOH / Citric acid | Neutralizer | q.s. pH 5.5 |
| F | Water | | 4.00% |
| | Sodium Benzoate | Preservative | 0.45% |
| | Trisodium Citrate | Buffer | 0.50% |
| G | Perlogen^{®} SF 117 (Clariant) (Aqua (and) Glycol Distearate (and) Laureth-4) | Pearlizer | 5.00% |
| | FD&C Violette 2 (Cosnaderm) (CI 60730) | Colorant | 0.002% |
| H | Sodium Chloride | Rheology Modifier | 2.00% |

### Example 7b - Clear SLES free shower gel

| Phase | Ingredient | Function | |
|---|---|---|---|
| A | Water | Diluent | 35.0% |
| | Sodium Coco Sulfate, 90% | Surfactant | 4.0% |
| B | Genagen^{®} CAB 818 (Clariant) (Cocamidopropyl Betaine) | Surfactant | 4.0% |
| | Nipaguard^{®} SCE (Clariant) (Sorbitan Caprylate (and) Propanediol (and) Benzoic Acid) | Preservative | 1.0% |
| C | GlucoTain^{®} Care (Clariant) (Cocoyl Methyl Glucamide) | Surfactant | 5.0% |
| D | Blend of Example 1 | Emollient | 5.0% |
| | Emulsogen^{®} DTC 070 (Clariant) (Trideceth-7 Carboxylic Acid) | Solvent | 10.0% |
| E | Genapol^{®} LT (Clariant) (PEG-150 Polyglyceryl-2 Tristearate (and) Laureth-3 (and) Dipropylene Glycol) | Thickener | 4.0% |
| F | Sodium Hydroxide, 10% Aqueous Solution | pH Adjuster | q.s. |
| G | Water | Solubiliser | q.s. |

### Example 7c - Sulfate free shower cream for atopic skin

| Phase | Ingredient | Function | |
|---|---|---|---|
| A | Blend of Example 1 | Emollient | 10.0% |
| | Stearic Acid | Stabilizer | 5.0% |
| | Native Coco Oil (Cocos Nucifera (Coconut) Oil) | Emollient | 5.0% |
| | Velsan^{®} SC (Clariant) (Sorbitan Caprylate) | Preservative Booster | 1.0% |
| | Lanette^{®} O (Cetearyl Alcohol) | Stabilizer | 1.8% |
| | Lanette^{®} 18 (Stearyl Alcohol) | Stabilizer | 1.2% |
| B | GlucoTain^{®} Plus (Clariant) (Capryloyl/Caproyl Methyl Glucamide (and) Lauroyl/Myristoyl Methyl Glucamide) | Co-Surfactant | 3.4% |
| | Genagen^{®} 3SB (Clariant) (Coco Betaine (and) Sodium Methyl Cocoyl Taurate (and) Sodium Cocoyl Isethionate) | Surfactant | 37.7% |
| | GlucoTain^{®} liquiFlex (Clariant) (Lauroyl/Myristoyl Methyl Glucamide (and) Coco-Betaine) | Co-Surfactant | 5.7% |
| | Genapol^{®} LA 030 (Clariant) (Laureth-3) | Rheology Modifier | 1.0% |
| | NaOH solution (25%) | Neutralizer | ∼0.3% |
| | Water | | ad 100% |
| C | NaOH solution (10%) | Neutralizer | 1.0% |
| | Glycerin 85% | Humectant | 2.5% |
| | Velsan^{®} AS (Clariant) (p-Anisic Acid) | Fragrance | 0.3% |
| D | Carbopol^{®} Ultrez 21 (Acrylates / C10-30 Alkyl Acrylate Crosspolymer) | Rheology Modifier | 0.3% |
| E | Niacinamide | Active | 2.0% |
| | Water | Diluent | 10.0% |
| F | Citric Acid solution (50%) | Neutralizing Agent | q.s. pH 5.5 |

### Example 7d - Nutritive hair mask

| Phase | Ingredient | Function | |
|---|---|---|---|
| A | Plantasens^{®} Natural Emulsifier CP5 (Clariant) (Glyceryl Oleate (and) Polyglyceryl-3-Polyricinoleate (and) Olea Europaea (Olive) Oil Unsaponifiables) | Emulsifier | 1.0 % |
| | Cetyl Alcohol | Stabilizer | 4.0 % |
| | Stearic Acid | Stabilizer | 1.0 % |
| | Plantasens^{®} Argan Butter (Clariant) (Argania Spinosa Kernel Oil (and) Hydrogenated Vegetable Oil) | Emollient | 1.0 % |
| | Plantasens^{®} Apricot Butter (Clariant) (Prunus Armeniaca (Apricot) Kernel Oil (and) Hydrogenated Vegetable Oil) | Emollient | 0.5 % |
| | Blend of Example 1 | Emollient | 1.4 % |
| B | Water | Diluent | ad 100 % |
| | Allantoin (Clariant) | Active | 0.1 % |
| | Tylose H100070 NP2 (Hydroxyethylcellulose) | Rheology Modifier | 0.5 % |
| | Genamin^{®} CTAC (Clariant) (Cetrimonium Chloride) | Conditioning Agent | 0.5 % |
| | Genamin^{®} KDMP (Clariant) (Behentrimonium Chloride) | Conditioning Agent | 1.5 % |
| | Glycerol | Humectant | 5.0 % |
| C | Beraclay Red (Beraca) (Magnesium Aluminum Silicate / Kaolin / Mica) | Absorbant | 2.0 % |
| | Beraclay Dark Red (Beraca) (Magnesium Aluminum Silicate / Kaolin / Montmorillonite / Mica) | Absorbant | 4.0 % |
| | ImerCare^{™} 04K (Kaolin) | Absorbant | 12.0 % |
| D | Plantasens^{®} Lime Serum (Clariant) (Citrus Limon (Lemon) Seed Oil (and) Phytosterols (and) Olea Europaea (Olive) Oil Unsaponifiables (and) Beeswax) | Active | 0.3 % |
| | Plantasens^{®} Olive LD (Clariant) (Hydrogenated Ethylhexyl Olivate (and) Hydrogenated Olive Oil Unsaponifiables) | Emollient | 0.5 % |
| | Fragrance 'Orchidee' (Cosnaderm) | Fragrance | 0.5 % |
| E | EMortal^{™} Pep (BioSpectrum) (Propanediol (and) Water (and) Pisum Sativum (Pea) Peptide) | Active | 0.8 % |
| | COSI-PLANT Green Tea GW (Cosnaderm) (Glycerin, Aqua, Camellia Sinensis Leaf Extract) | Active | 0.3 % |
| | Tocopheryl Acetate | Active | 0.5 % |
| F | Nipaguard^{®} SCE (Clariant) (Sorbitan Caprylate (and) Propanediol (and) Benzoic Acid) | Preservative | 1.2 % |
| | Sodium Hydroxide 10% | Neutralizing Agent | q.s. to pH approx. 4.3 |

### Example 8 - Sun care formulations

### Example 8a - Protective sun care oil SPF 30 (calculated)

| Phase | Ingredient | Function | |
|---|---|---|---|
| A | C12-15 Alkylbenzoate | Emollient | ad 100% |
| | Plantasens^{®} Carefeel Light (Clariant) (Ethylhexyl Olivate) | Emollient | 10.0% |
| | Blend of Example 1 | Emollient | 5.0% |
| | Dow Corning^{®} 246 (Cyclohexasiloxane (and) Cyclopentasiloxane) | Emollient | 13.0% |
| | Eclipsogen^{®} AVB (Clariant) (Butyl Methoxydibenzoylmethane) | UV Filter | 5.0% |
| | Eclipsogen^{®} OC (Clariant) (Octocrylene) | UV Filter | 7.0% |
| | Eclipsogen^{®} Sorb S (Clariant) (Bis-Ethylhexyloxyphenol Methoxyphenyl Triazine) | UV Filter | 2.0% |
| | Eclipsogen^{®} EHMC (Clariant) (Ethylhexyl Methoxycinnamate) | UV Filter | 7.0% |
| B | Ethanol | Solvent | 15.0% |
| C | Rainforest Annatto Oil (Beraca) (Bixa Orellana Annatto Oil) | Emollient | 1.0% |
| | Plantasens^{®} Abyssinian Oil (Clariant) (Crambe Abyssinica Seed Oil) | Emollient | 2.0% |
| | Tocopheryl Acetate | Antioxidant | 0.5% |
| | Fragrance "Frangipane Flower" | Fragrance | 0.3% |

### Example 8b - Sun care oil

| Phase | Ingredient | Function | |
|---|---|---|---|
| A | Tegosoft^{®} TN (C12-15 Alkyl Benzoate) | Emollient | ad 100 % |
| | Plantasens^{®} Carefeel Light (Clariant) (Ethylhexyl Olivate) | Emollient | 15.0 % |
| | Plantasens^{®} Flash 100 (Clariant) (Tridecane (and) Pentadecane) | Emollient | 13.0 % |
| | Eclipsogen^{®} AVB (Clariant) (Butyl Methoxydibenzoylmethane) | UV Filter | 5.0 % |
| | Eclipsogen^{®} OCR (Clariant) (Octocrylene) | UV Filter | 7.0 % |
| | Eclipsogen^{®} Sorb S (Clariant) (Bis-Ethylhexyloxylphenol Methoxyphenyl Triazine) | UV Filter | 2.0 % |
| | Eclipsogen^{®} EHMC (Clariant) (Ethylhexylmethoxycinnamate) | UV Filter | 7.0 % |
| B | Blend of Example 1 | Emollient | 2.0 % |
| | Tocopheryl Acetate | Antioxidant | 0.5 % |
| | Plantasens^{®}Olive Active HP (Clariant) (Olea Europaea (Olive) Oil Unsaponifiables) | Active | 2.0 % |
| | Fragrance "Kirschblüte" | Fragrance | 0.3 % |

### Example 8c - After sun cream gel

| Phase | Ingredient | Function | |
|---|---|---|---|
| A | Mineral Oil, low viscosity | Emollient | 3.0 % |
| | Isopropyl Palmitate | Emollient | 3.0 % |
| | Cetiol^{®} SN (Cetearyl Isononanoate) | Emollient | 3.0 % |
| | Blend of Example 1 | Emollient | 6.0 % |
| | Tocopheryl Acetate | Antioxidant | 1.0 % |
| B | Aristoflex^{®} AVC (Clariant) (Ammonium Acryloyldimethyltaurate/VP Copolymer) | Rheology Modifier | 1.0 % |
| C | Water | | ad 100 % |
| | Glycerin | Humectant | 3.0 % |
| | Allantoin^{®} | Active | 0.2 % |
| | Preservative | Preservative | q.s. |
| | Panthenol | Humectant | 1.0 % |
| D | Collagen nativ 1% | Active | 3.0 % |
| | Alcohol | Solvent | 1.5 % |
| | Fragrance | Fragrance | 0.3 % |

### Example 8d - Sun cream SPF 50+, PA++++ (calculated)

| Phase | Ingredient | Function | |
|---|---|---|---|
| A | Water | Diluent | ad 100% |
| | Glycerin | Humectant | 3.00% |
| | Eclipsogen^{®} PBSA (Clariant) (Phenylbenzimidazole Sulfonic Acid) | UV Filter | 2.00% |
| | Sodium Hydroxide | pH Adjuster | q.s. to pH 7 |
| B | Hostaphat^{®} KL 340 D (Clariant) (Trilaureth-4 Phosphate) | Emulsifier | 0.75% |
| | Cetearyl Alcohol | Stabilizer | 2.25% |
| | Plantasens^{®} Carefeel Light (Clariant) (Ethylhexyl Olivate) | Emollient | 4.00% |
| | Blend of Example 1 | Emollient | 2.00% |
| | Eclipsogen^{®} Sorb S (Clariant) (Bis-Ethylhexyloxyphenol Methoxyphenyl Triazine) | UV Filter | 5.00% |
| | Eclipsogen^{®} OC (Clariant) (Octocrylene) | UV Filter | 6.00% |
| | Eclipsogen^{®} EHT (Clariant) (Ethylhexyl Triazone) | UV Filter | 3.00% |
| C | Eclipsogen^{®} Sorb M (Clariant) (Methylene Bis-Benzotriazolyl Tetramethylbutylphenol (and) Water (and) Decyl Glucoside (and) Xanthan Gum (and) Propylene Glycol) | UV Filter | 10.00% |
| D | Aristoflex^{®} Silk (Clariant) (Sodium Polyacryloyldimethyl Taurate) | Rheology Modifier | 1.50% |
| E | Nipaguard^{®} PO5 (Clariant) (Phenoxyethanol (and) Piroctone Olamine) | Preservative | 1.00% |
| | Perfume | Fragrance | 0.30% |

### Example 9 - Solid bar formulations

### Example 9a - Facial cleanser

| Ingredient | INCI | Function | % |
|---|---|---|---|
| Tapioca Starch | Tapioca Starch | Vehicle | 63 |
| Blend of Example 1 | | Sensory / Active | 2 |
| Hostapon^{®} TPHC | Sodium Methyl Oleoyl Taurate | Surfactant | 8 |
| Hostapon^{®} SCI 85P | Sodium Cocoyl Isethionate | Surfactant | 23 |
| Amazonian White Clay | Kaolin | Sensory | 4 |

### Example 9b - Solid sun care cream

| Phase | Ingredient | INCI | Function | % |
|---|---|---|---|---|
| A | Plantasens^{®} Olive LD | Hydrogenated Ethylhexyl Olivate; Hydrogenated Olive Oil Unsaponifiables | Emollient | 4 |
| | Plantasens^{®} Carefeel Light | Ethylhexyl Olivate | Emollient | 5.45 |
| | Blend of Example 1 | | Emollient | 3 |
| | Hostacerin^{®} DGI | Polyglyceryl-2 Sesquiisostearate | Structure Former, Sensory | 3 |
| B | Plantasens^{®} Cosmetic Wax A4 | Hydrogenated Vegetable Oil | Structure Former | 15.75 |
| | Microcrystalline Wax | Microcrystalline Wax | Structure Former | 6.5 |
| | Stearic Acid | Stearic Acid | Structure Former | 9 |
| | Myristic Acid | Myristic Acid | Structure Former | 6 |
| | Plantasens^{®} Emulsifier HP30 | Glyceryl Stearate (and) Cetearyl Alcohol (and) Sodium Stearoyl Lactylate | Structure Former, Sensory | 2.3 |
| | Tapioca Starch | Tapioca Starch | Sensory | 19.5 |
| C | Jeescreen^{®} OS | Octisalate | UV Filter | 5 |
| | Homosalate | Homosalate | UV Filter | 10 |
| | Eclipsogen^{®} AVB | Avobenzone | UV Filter | 3 |
| | Jeescreen^{®} OC | Octocrylene | UV Filter | 7.5 |

### Example 9c - Solid cream

| Ingredient | INCI | Function | % |
|---|---|---|---|
| Behenyl Alcohol | Behenyl Alcohol | Structuring agent | 6 |
| Plantasens^{®} Cosmetic Wax A4 | Hydrogenated Vegetable Oil | Structuring agent | 18 |
| Plantasens^{®} Flash 100 | Tridecane (and) Pentadecane | Emollient / Conditioner | 6 |
| Plantasens^{®} Olive LD | Hydrogenated Ethylhexyl Olivate (and) Hydrogenated Olive Oil Unsaponifiables | Emollient / Conditioner | 15.5 |
| Plantasens^{®} Carefeel Light | Ethylhexyl Olivate | Emollient / Conditioner | 15.5 |
| Blend of Example 1 | | Emollient / Conditioner | 3 |
| Tapioca Starch | Tapioca Starch | Sensory | 36 |

### Example 9d - Solid shampoo

| Ingredient | INCI | Function | % |
|---|---|---|---|
| Tapioca Starch | Tapioca Starch | Vehicle | 60 |
| Genagen^{®} KB | Coco-Betaine | Surfactant / Sensory | 5 |
| Blend of Example 1 | | Emollient | 3 |
| Genadvance^{®} Hydra | Lauryl/Myristyl Polyricinoleate; Glycerin | Emollient / Humectant | 2 |
| Hostapon^{®} TPHC | Sodium Methyl Oleoyl Taurate | Surfactant | 4 |
| Hostapon^{®} SCI 85P | Sodium Cocoyl Isethionate | Surfactant | 20 |
| Amazonian White Clay | Kaolin | Sensory | 4 |
| Natural Aromatic Extract | Aromatic Coconut Extract | Aromatic Extract / Fragrance | 2 |

### Example 9e - Solid conditioner

| Ingredient | INCI | Function | % |
|---|---|---|---|
| Tapioca Starch | Tapioca Starch | Vehicle | 70 |
| Genadvance^{®} Repair | Quaternium-98 | Conditioner / Active | 10 |
| Natural Aromatic Extract | Grean Tea Mint Extract | Aromatic Extract / Fragrance | 2 |
| Hostapon^{®} TPHC | Sodium Methyl Oleoyl Taurate | Surfactant | 3 |
| Blend of Example 1 | | Emollient | 2 |
| Plantasens^{®} Olive LD | Hydrogenated Ethylhexyl Olivate; Hydrogenated Olive Oil Unsaponifiables | Emollient | 4 |
| Beraca Green Clay | Kaolin | Sensory | 5 |
| DL-Panthenol 50L (Liquid) | DL-Panthenol | Emollient | 4 |

### Example 9f - Solid cleansing body bar

| Phase | Ingredient | INCI | Function | % |
|---|---|---|---|---|
| A | Plantasens^{®} Crystolive Wax | Olea Europaea (Olive) Oil Unsaponifiables | Consistency agent | 9.5 |
| | Plantasens^{®} Cosmetic Wax A5 | Hydrogenated Vegetable Oil | Consistency agent | 5.5 |
| | Cetyl Alcohol | Cetyl Alcohol | Structuring agent | 21.5 |
| | Refined Murumuru Butter | Astrocaryum Murumuru Seed Butter (and) Tocopherol | Emollient / Moisturizer | 14.5 |
| | Blend of Example 1 | | Emollient | 6 |
| B | Plantasens^{®} Emulsifier SFO | Sunflower Seed Oil Sorbitol Esters | Emulsifier | 3 |
| | Plantasens^{®} Emulsifier HP 30 | Glyceryl Stearate (and) Cetearyl Alcohol (and) Sodium Stearoyl Lactylate | Emulsifier | 3 |
| C | Amazonian White Clay | Kaolin | Light Exfoliating agent + Colorant | 6.5 |
| | Tapioca Starch | Tapioca Starch | Sensory agent | 9 |
| D | Hostapon^{®} CCG | Sodium Cocoyl Glutamate | Surfactant | 15 |
| E | Quartz Crystal Facial | Silica | Exfoliating agent | 2 |
| F | MultiEx BSASM^{™} | Butylene Glycol (and) Water (and) Centella Asiatica Extract (and) Polygonum Cuspidatum Root Extract (and) Scutellaria Baicalensis Root Extract (and) Camellia Sinensis Leaf Extract (and) Glycyrrhiza Glabra (Licorice) Root Extract (and) Chamomilla Recutita (Matricaria) Flower Extract (and) Rosmarinus Officinalis (Rosemary) Leaf Extract | Active ingredient | 2 |
| | BioDTox^{®} | Propanediol (and) Aloe Barbadensis Leaf Extract (and) Bioflavonoids (and) Brassica Oleracea Italica (Broccoli) Extract | Active ingredient | 2 |
| | Vanilla Extract | Vanilla Extract | Fragrance | 0.5 |

### Example 9g - Solid hair stick

| Ingredient | INCI | Function | % |
|---|---|---|---|
| Plantasens^{®} Crystolive Wax | Olea Europaea (Olive) Oil Unsaponifiables | Structuring agent / Viscosifier | 15 |
| Plantasens^{®} Cosmetic Wax A5 | Hydrogenated Vegetable Oil | Structuring agent | 20 |
| Cetearyl Alcohol | Cetearyl Alcohol | Thickener / Stabilizer | 10 |
| Genamin^{®} BTMS | Behentrimonium Methosulfate | Conditioning agent | 11.5 |
| Plantasens^{®} Olive LD | Hydrogenated Olive Oil Unsaponifiables | | 7 |
| Plantasens^{®} Flash 80 | Dodecane | Emollient | 10 |
| Blend of Example 1 | | Emollient | 5 |
| Plantasens^{®} VP 170 | Ricinus Communis (Castor) Seed Oil (and) Hydrogenated Castor Oil (and) Copernicia Cerifera (Carnauba) Wax (and) Cera Alba (Beeswax) | Emollient | 10 |
| Plantasens^{®} VL | Butyrospermum Parkii (Shea) Butter (and) Glyceryl Rosinate (and) Olea Europaea (Olive) Oil Unsaponifiables | Emollient | 5 |
| Dongbaek Oil | Camellia Japonica Seed Oil | Active ingredient | 4 |
| Nipaguard^{®} SCA | Sorbitan Caprylate (and) Benzyl Alcohol | Preservative | 1.5 |
| Nipanox^{™} BHT | Butylated Hydroxy Toluene | Antioxidant | 0.3 |
| Perfume | Fragrance | Fragrance | 0.7 |

### Example 9h - Solid shampoo - plastic free

| Ingredient | INCI | Function | % |
|---|---|---|---|
| Medialan^{®} LD PF 10 | Sodium Lauroyl Sarcosinate | Surfactant | 10 |
| Hostapon^{®} SCI 85 G | Sodium Cocoyl Isethionate | Surfactant | 37 |
| Genagen^{®} KB | Coco-Betaine | Surfactant | 20 |
| Polyglykol 8000 | PEG-180 | Binder | 27 |
| Genadvance^{®} Repair | Quaternium-98 | Hair Conditioning agent | 3 |
| Blend of Example 1 | | Emollient | 1.5 |
| Nipaguard^{®} SCE | Sorbitan Caprylate (and) Propanediol (and) Benzoic Acid | Preservative | 1 |
| Perfume | Fragrance | Fragrance | 0.5 |

### Example 9i - Body odour deodorizing soap bar

| Ingredient | INCI | Function | % |
|---|---|---|---|
| Soap Base Opal 508 | Sodium Palmate, Sodium Palm Kernelate, Water, Glycerin, Pentasodium Pentatate, EDTA | Surfactant base | ad 100 |
| Blend of Example 1 | | Emollient | 2 |
| Caremag^{™} D | Magnesium Hydroxide (and) Magnesium Carbonate Hydroxide | Deodorant agent, Texturizer, Antimicrobial agent | 2.5 |
| Velsan^{®} CGE | Caprylyl Glyceryl Ether | Emollient | 1 |
| Perfume | Fragrance | | 0.4 |
| Color | Dye | | 0.05 |

### Example 9j - Conditioning soap

| Ingredient | INCI | Function | % |
|---|---|---|---|
| Soap Base CremerSap BV 422 | Sodium Olivate, Water, Sodium Chloride | Surfactant base | ad 100 |
| Genadvance^{®} Hydra | Lauryl/Myristyl Polyricinoleate (and) Glycerin | Hair Conditioning agent | 5 |
| Blend of Example 1 | | Emollient | 2 |
| Perfume | Fragrance | | 0.1 |

### Example 9k - Caring soap

| Ingredient | INCI | Function | % |
|---|---|---|---|
| Soap Base Opal 508-SG | Sodium Palmate, Sodium Palm Kernelate, Water, Glycerin, Pentasodium Pentatate, EDTA | Surfactant base | ad 100 |
| Blend of Example 1 | | Emollient | 3 |
| Fragrance Flovea (Cosnaderm) | Fragrance | | 0.2 |
| Cosnacolor FD&C Red No4 Cl 14700 | Dye | | 0.01 |

### Example 9l - Mineral based deodorant

| Phase | Ingredient | INCI | Function | % |
|---|---|---|---|---|
| A | Caremag^{™} D | Magnesium Hydroxide, Magnesium Carbonate Hydroxide | Deodorant agent, Texturizer, Antimicrobial agent | ad 100 |
| | MadeWhite^{™} | Water (and) Pentylene Glycol (and) Madecassoside | Brightening agent | 2 |
| | Tocopheryl Acetate | Tocopheryl Acetate | | 2 |
| | Tapioca Starch | Tapioca Starch | Texturizer | 12 |
| | Jasmine Oil Fragrance | Fragrance | | 0.5 |
| | Octopirox^{®} | Piroctone Olamine | Antimicrobial | 0.1 |
| B | Plantasens^{®} Flash 80 | Dodecane | Emollient | 20 |
| | Blend of Example 1 | | Emollient | 5 |
| | Plantasens^{®} Refined Babassu Butter | Orbygnya Oleifera (Babassu) Seed Oil | Emollient | 3.4 |
| | Plantasens^{®} Olive LD | Hydrogenated Ethylhexyl Olivate (and) Hydrogenated Olive Oil Unsaponifables | Emollient | 5 |
| | Plantasens^{®} Cosmetic Wax A4 | Hydrogenated Vegetable Oil | Structuring agent | 15 |
| | Candellila wax | Candellila wax | Structuring agent | 15 |

### Example 10 - Hand hygiene formulations

### Example 10a - Hand hygiene cream gel

| Phase | Ingredient | INCI | Function | w/w% |
|---|---|---|---|---|
| A | Water | Aqua | Solvent | To 100 |
| | Ethanol | Ethanol | Solvent, Disinfectant | 70 |
| | Genapol^{®} G260 | Glycereth-26 | Moisturization agent | 3 |
| B | Aristoflex^{®} Velvet | Polyacrylate crosspolymer-11 | Thickener, Sensory Enhancer | 1 |
| C | Blend of Example 1 | | Emollient | 1 |
| | Fragrance | | Fragrance | qs |
| D | JM Acticare^{™} | Silver Chloride, Titanium Dioxide, Diethylhexyl sodium sulfosuccinate, Propylene glycol | Antimicrobial, Disinfectant | 0.1 |

### Example 10b - Hand hygiene wipes

| Phase | Ingredient | INCI | w/w% |
|---|---|---|---|
| A | Water | Aqua | To 100 |
| | Polyglykol 400 | PEG-8 | 2 |
| | Genapol^{®} G260 | Glycereth-26 | 2 |
| B | GlucoTain^{®} Sense | Sunfloweroyl methyl glucamide | 0.5 |
| | Emulsogen^{®} HCO 040 | PEG-40 Hydrogenated castor oil | 1 |
| | Blend of Example 1 | | 1 |
| | Fragrance | | 0.2 |
| C | Benzethonium Chloride | Benzethonium Chloride | 0.1 |

### Example 11 - Facial cleansing wipes

| Phase | Ingredient | INCI | % (w/w) |
|---|---|---|---|
| A | Water | Water | To 100 |
| A | Panthenol | Panthenol | 0.1 |
| A | Aristoflex^{®} TAC | Ammonium Acrylyol Dimethyl Taurate/ Carboxyethyl Acrylate Crosspolymer | 0.1 |
| B | Plantasens^{®} Emulsifier CCT | Caprylic/Capric Triglyceride, Aqua, Lauryl Glucoside, Glycerin, Sodium Lauroyl Lactylate, Glyceryl Stearate, Cetearyl Alcohol, Sodium Stearoyl Lactylate | 2.5 |
| B | Blend of Example 1 | | 5 |
| B | Tocopheryl Acetate | Tocopheryl Acetate | 0.05 |
| B | Nipaguard^{®} SCE | Sorbitan Caprylate, Propanediol, Benzoic Acid | 1.5 |
| C | Fragrance | Fragrance | 2 |

### Example 12 - Skin and hair leave-on formulations

### Example 12a - Hair and skin moisturizing nectar

| Phase | Ingredient | Function | % |
|---|---|---|---|
| A | Water | Solvent | ad 100 |
| B | Blend of Example 1 | Skin Conditioning Agent | 5.0 |
| | Dongbaek (Tsubaki) Oil (Camellia Japonica Seed Oil) | Anti-Ageing Active | 2.0 |
| | Genadvance^{®} Hydra (Lauryl/Myristyl Polyricinoleate (and) Glycerin) | Hair Moisturizer, Hair Conditioner | 1.5 |
| C | Aristoflex^{®} Silk (Sodium Polyacryloyldimethyl Taurate) | Rheology Modifier | 0.7 |
| D | Herbex^{™} Yeast Beta Glucan (Butylene Glycol, Yeast Beta Glucan) | Skin Well-Being Enhancer, UV Protector | 2.0 |
| | Plantasens^{®} Berto^{™} Orthomoist (Orthosiphon Stamineus Leaf Extract (and) Chlorphenesin) | Skin Moisturizer | 1.0 |
| E | Nipaguard^{®} EHP (Ethylhexylglycerin, Phenoxyethanol) | Preservative | 1.0 |
| | Fragrance | Fragrance | 0.4 |
| F | Sodium Hydroxide 10% soln. (pH: 4,5-5,5) | pH Adjuster | q.s. |

### Example 12b - Skin and hair nourishing cream

| Phase | Ingredient | Function | % |
|---|---|---|---|
| A | Water | Solvent | ad 100 |
| B | Blend of Example 1 | Skin Conditioning Agent | 3.0 |
| | Plantasens^{®} Sunflower Butter (Helianthus Annuus (Sunflower) Seed Oil (and) Hydrogenated Vegetable Oil) | Film Former | 4.0 |
| | Plantasens^{®} Emulsifier HP 10 (Sucrose Polystearate, Cetearyl Alcohol, Olea Europaea (Olive) Oil Unsaponifiables) | Emulsifier | 3.0 |
| | Genadvance^{®} Hydra (Lauryl/Myristyl Polyricinoleate (and) Glycerin) | Hair Moisturizer, Hair Conditioner | 1.5 |
| C | Aristoflex^{®} BLV (Ammonium Acryloyldimethyltaurate/ Beheneth-25 Methacrylate Crosspolymer) | Rheology Modifier | 0.7 |
| | Aristoflex^{®} AVC (Ammonium Acryloyldimethyltaurate/ VP Copolymer) | Rheology Modifier | 0.3 |
| D | Herbex^{™} Yeast Beta Glucan (Butylene Glycol, Yeast Beta Glucan) | Skin Well-Being Enhancer, UV Protector | 2.0 |
| | RedSnow^{®} (Propanediol, Water, Camellia Japonica Flower Extract) | Anti-Ageing Active | 2.0 |
| | Plantasens^{®} Berto^{™} Orthomoist (Orthosiphon Stamineus Leaf Extract (and) Chlorphenesin) | Skin Moisturizer | 1.0 |
| E | Nipaguard^{®} EHP (Ethylhexylglycerin, Phenoxyethanol) | Preservative | 1.0 |
| | Fragrance | Fragrance | 0.4 |
| F | Sodium Hydroxide 10% soln. (pH: 4,5-5,5) | pH Adjuster | q.s. |

### Example 13 - Make up

### Example 13a - Blemish base (BB) cream formulation

| Phase | Ingredient | % |
|---|---|---|
| A | Aqua | to 100 |
| | Glycerin | 3.00 |
| B | Hostaphat^{®} KL 340 D (Trilaureth-4-Phosphate) | 2.2 |
| | Caprylic/Capric Triglyceride | 2.0 |
| | Plantasens^{®} Olive Active HP (Olea Europaea (Olive) Oil Unsaponifiables) | 1.0 |
| | Plantasens^{®} Carefeel Comfort (Macadamia Integrifolia/Tetraphylla Seed Oil (and) Crambe Abyssinica Seed Oil (and) Orbignya Oleifera Seed Oil) | 1.0 |
| | Plantasens^{®} Abyssinian Oil (Crambe Abyssinica Seed Oil) | 1.0 |
| | Blend of Example 1 | 1.0 |
| | Dicaprylyl Ether | 1.0 |
| | Plantasens^{®} Olive LD (Hydrogenated Ethylhexyl Olivate (and) Hydrogenated Olive Oil Unsaponifiables) | 2.0 |
| | Dodecane | 3.0 |
| | Aristoflex^{®} Velvet (Polyacrylate Crosspolymer-11) | 0.8 |
| | Aristoflex^{®} AVC (Ammonium Acryloyldimethyltaurate/ VP Copolymer) | 0.8 |
| | Caprylyl Methicone (and) PEG-12 Dimethicone/PPG-20 Crosspolymer | 3.0 |
| | Plantasens^{®} Vitamin E Natural 50 (Tocopherol and Helianthus annuus (Sunflower) Seed oil) | 0.5 |
| C | CI77891 (and) aqua (and) glycerin (and) xanthan gum (and) sodium citrate | 11.0 |
| | CI77492 (and) aqua (and) glycerin (and) xanthan gum (and) sodium citrate | 1.6 |
| | CI77491 (and) aqua (and) glycerin (and) xanthan gum (and) sodium citrate | 0.7 |
| | CI77499 (and) aqua (and) glycerin (and) xanthan gum (and) sodium citrate | 0.2 |
| D | Preservative | 1.0 |
| E | Perfume | 0.3 |

### Example 13b - Volumising mascara emulsion formula

| Phase | Ingredient | % |
|---|---|---|
| A | Aqua | to 100 |
| | Disodium EDTA | 0.1 |
| | Propylene Glycol | 2.0 |
| | Triethanolamine | 1.6 |
| A1 | Hydroxyethylcellulose | 0.2 |
| A2 | Magnesium Aluminium Silicate | 1.4 |
| B | Isostearic acid | 1.0 |
| | Phenylpropyldimethylsiloxylicate | 3.0 |
| | Stearic Acid | 3.0 |
| | Glyceryl Stearate SE | 1.2 |
| | Polyvinyl Stearyl Ether | 5.0 |
| | Copernicia Prunifera (Carnauba) Wax | 3.0 |
| | Candelilla Wax | 4.0 |
| | Blend of Example 1 | 0.5 |
| | Synthetic Beeswax | 4.0 |
| C | Cl 77499, Methicone | 10.0 |
| D | Phenoxetol^{®} (Phenoxyethanol) | 0.6 |
| | O-Cymen-5-ol | 0.1 |
| E | Hydrolyzed Corn Protein, Hydrolyzed Wheat Protein, Hydrolyzed Soy Protein | 2.0 |

### Example 13c - Gelee glow multi use highlighter

| Phase | Ingredient | % |
|---|---|---|
| A | Aqua | to 100 |
| | Glycerin | 10.00 |
| | Carrageenan | 0.75 |
| | Xanthan Gum | 0.25 |
| B | Plantasens^{®} Emulsifier HP10 (Sucrose Polystearate (and) Cetearyl Alcohol (and) Olea Europaea (Olive) Oil Unsaponifiables) | 1.50 |
| | Blend of Example 1 | 2.00 |
| | Plantasens^{®} Carefeel Light (Ethylhexyl Oleate) | 2.00 |
| | Plantasens^{®} Olive LD SP ECO (Coco-Caprylate/Caprate (and) Hydrogenated Olive Oil Unsaponifiables) | 1.00 |
| C | Mica | 6.50 |
| | Mica (and) Titanium Dioxide (and) Iron Oxides | 3.00 |
| | Synthetic Fluorphlogopite (and) Cl 77891 (and) Tin Oxide | 3.00 |
| | Propanediol | 20.00 |
| D | Nipaguard^{®} SCE (Sorbitan Caprylate (and) Propanediol (and) Benzoic Acid) | 1.00 |
| | Eosidin^{™} (Propanediol (and) Water (and) Citrus Unshiu Peel Extract) | 1.00 |
| | Perfume | 0.20 |

### Example 14 - Body and face leave-on formulations

### Example 14a - Body lotion for dry skin

| Phase | Ingredient | % |
|---|---|---|
| A | Decyl Oleate | 2.0 |
| | Plantasens^{®} CP5 (Glyceryl Oleate (and) Polyglyceryl-3 Polyricinoleate (and) Olea Europaea (Olive) Oil Unsaponifiables) | 5.0 |
| | Blend of Example 1 | 5.0 |
| | Plantasens^{®} Olive LD (Hydrogenated Ethylhexyl Olivate (and) Hydrogenated Olive Oil Unsaponifiables) | 2.0 |
| | Caprylic/Capric Triglyceride | 5.0 |
| B | Glycerin | 3.0 |
| | Xanthan gum | 0.2 |
| C | Water | up to 100% |
| D | Hostapon^{®} CGN (Sodium Cocoyl Glutamate) | 1.0 |
| E | Sodium Hydroxide (10%) | qs pH 7.0 |
| F | Aristoflex^{®} Velvet (Polyacrylate Crosspolymer-11) | 1.0 |
| G | Nipaguard^{®} SCE (Sorbitan Caprylate and Propanediol and Benzoic Acid) | 1.4 |
| H | Fragrance | 1 |
| I | Citric acid (10%) | qs pH 5.0-5.2 |

### Example 14b - Smooth moisturiser for body

| Phase | Ingredient | % |
|---|---|---|
| A | Water | up to 100% |
| | Glycerin | 3.5 |
| B | Aristoflex^{®} AVC (Ammonium Acryloyl Dimethyltaurate/VP Copolymer) | 0.8 |
| C | Plantasens^{®} Olive LD (Hydrogenated Ethylhexyl Olivate (and) Hydrogenated Olive Oil Unsaponifiables) | 5.0 |
| | Caprylic/Capric Triglyceride | 5.0 |
| | Blend of Example 1 | 5.0 |
| | Cetearyl alcohol | 1.0 |
| | Hostaphat^{®} KW 340 D (Triceteareth-4 Phosphate) | 4.0 |
| D | Tocopheryl Acetate | 0.3 |
| | Nipaguard^{®} PO5 (Phenoxyethanol and Piroctone Olamine) | 1.0 |
| | Fragrance | 1.0 |
| E | Citric acid (10%) | qs pH 4.5-5.0 |

### Example 14c - Ultra moisture night cream

| Phase | Ingredient | % |
|---|---|---|
| A | Caprylic/Capric Triglyceride | 3.0 |
| | Plantasens^{®} Olive LD (Hydrogenated Ethylhexyl Olivate (and) Hydrogenated Olive Oil Unsaponifiables) | 5.0 |
| | Plantasens^{®} Olive Squalane (Squalane) | 3.0 |
| | Blend of Example 1 | 5.0 |
| | Isopropyl Palmitate | 3.0 |
| | Plantasens^{®} Emulsifier CP40 (Polyglyceryl-3 Polyricinoleate and Sorbitan Oleate) | 5.0 |
| B | Water | up to 100% |
| | Glycerin | 3.0 |
| | Magnesium Sulfate 7 H2O | 0.8 |
| C | Tocopheryl Acetate | 0.2 |
| | Nipaguard^{®} SCP (Sorbitan Caprylate and Phenoxyethanol) | 1.4 |
| | Fragrance | 0.3 |

### Example 14d - Proage elixir - face oil

| Phase | Ingredient | % |
|---|---|---|
| A | Plantasens^{®} Abyssinian Oil (Crambe Abyssinica Seed Oil) | up to 100% |
| | Plantasens^{®} Olive Oil (Olea Europaea (Olive) Oil) | 23.25 |
| | Blend of Example 1 | 10.00 |
| | Plantasens^{®} Abyssinian Serum (Crambe Abyssinica Seed Oil (and) Phytosterols (and) Olea Europaea (Olive) Oil Unsaponifiables (and) Cera Alba (Beeswax)) | 0.10 |
| | Plantasens^{®} Olive Active HP (Olea Europaea (Olive) Oil Unsaponifiables) | 1.00 |
| | Rainforest Acai Oil (Euterpe Oleracea (Acai) Fruit Oil) | 1.00 |
| | Tocopheryl Acetate | 0.50 |
| | Cl 42053 | 1.35 |

### Example 14e - Daily moisturiser for men's care

| Phase | Ingredient | % |
|---|---|---|
| A | Dicaprylyl Ether | 2.50 |
| | Plantasens^{®} Olive LD (Hydrogenated Ethylhexyl Olivate (and) Hydrogenated Olive Oil Unsaponifiables) | 3.00 |
| | Plantasens^{®} Olive Active HP (Olea Europaea (Olive) Oil Unsaponifiables) | 1.00 |
| | Blend of Example 1 | 1.00 |
| | Cetearyl Ethylhexanoate | 2.50 |
| | Plantasens^{®} Emulsifier HP10 (Sucrose Polystearate (and) Cetearyl Alcohol (and) Olea Europaea (Olive) Oil Unsaponifiables) | 3.00 |
| B | Water | up to 100% |
| | Glycerin | 5.00 |
| C | Aristoflex^{®} AVC (Ammonium Acryloyldimethyltaurate/VP Copolymer) | 0.40 |
| | Aristoflex^{®} Velvet (Polyacrylate Crosspolymer-11) | 0.40 |
| D | Tocopheryl Acetate | 0.20 |
| | Nipaguard^{®} POB (Phenoxyethanol (and) Benzoic Acid (and) Piroctone Olamine) | 0.80 |
| | Fragrance | 0.20 |
| | Cl 42090 | 0.01 |
| E | Sodium Hydroxide solution (10%) | qs pH 5.0-5.3 |

### Example 14f - Soothing overnight face mask

| Phase | Ingredient | % |
|---|---|---|
| A | Water | up to 100% |
| | Rose Water (Rose Damascena Flower Water) | 5.0 |
| | Glycerin | 4.0 |
| | Beraca Kaolin Clay Light Green (Kaolin) | 0.5 |
| B | Plantasens^{®} Olive LD (Hydrogenated Ethylhexyl Olivate (and) Hydrogenated Olive Oil Unsaponifiables) | 5.0 |
| | Plantasens^{®} Abyssinian Oil (Crambe Abyssinica Seed Oil) | 5.0 |
| | Blend of Example 1 | 3.0 |
| | Plantasens^{®} Emulsifier HP30 (Glyceryl Stearate (and) Cetearyl Alcohol (and) Sodium Stearoyl Lactylate) | 3.0 |
| | Plantasens^{®} Emulsifier DGDS (Polyglyceryl-2 Distearate) | 5.0 |
| | Plantasens^{®} Babassu Butter Refined (Orbignya Oleifera Seed Oil) | 3.0 |
| | Plantasens^{®} Cosmetic Wax A5 (Hydrogenated Vegetable Oil) | 1.0 |
| | Plantasens^{®} Lemon Serum (Citrus Limon (Lemon) Seed Oil (and) Phytosterols (and) Olea Europaea (Olive) Oil Unsaponifiables (and) Cera Alba (Beeswax)) | 2.0 |
| | Stearic Acid | 1.0 |
| C | MultiEx BSASM^{™} (Butylene Glycol (and) Aqua (and) Centella Asiatica Extract (and) Polygonum Cuspidatum Root Extract (and) Scutellaria Baicalensis Root Extract (and) Camellia Sinensis Leaf Extract (and) Glycyrrhiza Glabra (Licorice) Root Extract (and) Chamomilla Recutita (Matricaria) Flower Extract (and) Rosmarinus Officinalis (Rosemary) Leaf Extract) | 3.0 |
| | HerbEx^{™} Mulberry Extract (Butylene Glycol (and) Aqua (and) Morus Alba Leaf Extract) | 2.0 |
| | HerbEx^{™} Tomato Extract (Glycerin (and) Aqua (and) Solanum Lycopersicum Fruit Extract) | 2.0 |
| | B-Circadin^{®} (Propanediol (and) Water (and) Lespedeza Capitata Leaf/Stem Extract) | 2.0 |
| D | Beraca Passion Fruit Oil (Passiflora Edulis Seed Oil (and) Tocopherol) | 2.0 |
| | Plantasens^{®} Olive Active HP (Olea Europaea (Olive) Oil Unsaponifiables) | 3.0 |
| | Nipaguard^{®} EHP (Phenoxyethanol (and) Ethylhexylglycerin) | 1.0 |
| | Cl 75810 | 0.4 |
| | Tocopheryl Acetate | 0.3 |
| | Perfume | 0.2 |
| E | Citric acid (10%) | qs pH 4.5-5.0 |

### Example 15 - Mild and effective deodorant roll on

| Phase | Ingredient | % |
|---|---|---|
| A | Water | up to 100% |
| | Xanthan gum | 0.5 |
| | Aristoflex^{®} Silk (Sodium Polyacryloyldimethyl Taurate) | 0.3 |
| | GlucoTain^{®} Sense (Sunfloweroyl Methylglucamide) | 2.0 |
| | Blend of Example 1 | 0.5 |
| | Velsan^{®} CGE (Caprylyl Glyceryl Ether) | 1.0 |
| | Fragrance | 1.0 |
| B | Water | 20.0 |
| | CareMag^{™} D (Magnesium Hydroxide (and) Magnesium Hydroxide Carbonate) | 7.5 |

## Claims

1. A composition comprising:
(A) 20 to 70% by weight of one or more oils comprising at least 75% by weight, referred to the total weight of the one or more oils, of one or more triglycerides as component A;
(B) 10 to 50% by weight of jojoba oil as component B;
(C) 5 to 30% by weight of one or more free fatty acids or salts thereof as component C;
(D) 5 to 25% by weight of squalene as component D; and
(E) 0 to 10% by weight of one or more antioxidants as component E.

2. The composition according to claim 1, wherein component A comprises at least 80% by weight, referred to the total weight of component A, of olive oil.

3. The composition according to claim 1 or 2, wherein component A comprises from 1 to 20% by weight, referred to the total weight of component A, of sunflower oil.

4. The composition according to any of claims 1 to 3, wherein the one or more free fatty acids or salts thereof of component C comprise at least 75% by weight, referred to the total weight of component C, of oleic acid or a salt thereof.

5. The composition according to any of claims 1 to 4, wherein component D is phytosqualene.

6. The composition according to any of claims 1 to 5, wherein the one or more antioxidants of component E comprise tocopherol.

7. The composition according to any of claims 1 to 6, wherein the composition comprises or consists of:
(A) 35 to 50% by weight of component A;
(B) 20 to 30% by weight of component B;
(C) 10 to 20% by weight of component C;
(D) 10 to 15% by weight of component D; and
(E) 0 to 5% by weight, preferably 1 to 5% by weight, of component E.

8. The composition according to any of claims 1 to 7, wherein the composition comprises or consists of:
(A) 40 to 50% by weight of one or more oils comprising at least 90% by weight, referred to the total weight of component A, of olive oil as component A;
(B) 20 to 30% by weight of jojoba oil as component B;
(C) 10 to 20% by weight of oleic acid as component C;
(D) 10 to 15% by weight of squalene as component D; and
(E) 1 to 4% by weight of tocopherol as component E.

9. The composition according to any of claims 1 to 8, wherein at least components A, B, C and D, and optionally component E, if present, are of plant origin.

10. A formulation comprising:
(I) 0.1 to 50% by weight of a composition as defined in any of claims 1 to 9; and
(II) 50 to 99.9% by weight of one or more cosmetically acceptable ingredients.

11. The formulation according to claim 10, wherein the formulation is a cosmetic formulation selected from the group consisting of skin care oil, skin care cream, skin care lotion, ointment, skin conditioner, face spray, body spray, hand oil, shower bath, hair conditioner, shaving gel, shampoo, body wash, facial cleanser, face mask, bubble bath, intimate wash, bath oil, cleansing milk, micellar water, make-up remover, cleansing wipes, hair mask, perfume, liquid soap, shaving soap, shaving stick, shaving foam, cleansing foam, day cream, anti-ageing cream, body milk, body lotion, body mousse, face serum, eye cream, sunscreen lotion, sun cream, face cream, after-shave lotion, pre-shaving cream, depilatory cream, skin-whitening gel, self-tanning cream, anti-acne gel, mascara, foundation, primer, concealer, blush, bronzer, blemish balm (bb) cream, eyeliner, night cream, eye brow gel, highlighter, lip stain, hand sanitizer, hair oil, nail varnish remover, conditioner, hair styling gel, hair styling cream, anti-frizz serum, scalp treatment, hair colorant, split end fluid, deodorant, antiperspirant, baby cream, insect repellent, hand cream, sunscreen gel, foot cream, exfoliator, body scrub, cellulite treatment, bar soap, cuticle cream, lip balm, hair treatment, eye shadow, bath additive, body mist, eau de toilette, mouthwash, toothpaste, lubricating gel, moisturizer, serum, toner, aqua sorbet, cream gel, styling mousse, dry shampoo, lip stick, lip gloss, body oil, shower milk, illuminator, lip crayon, hair spray, combing cream, and sunblock.

12. Use of a composition as defined in any of claims 1 to 9 as a skin conditioning agent.

13. The use according to claim 12, wherein the skin conditioning agent is an emollient, lubricant, moisturizer, refatting agent, smoothing agent and/or soothing agent.
